# EUROPEAN PATENT SPECIFICATION

(11) **EP 2 736 918 B1**
(45) Date of publication and mention of the grant of the patent: **29.08.2018**
(21) Application number: 12748307.1
(22) Date of filing: 30.07.2012
(51) Int. Cl.: C07J 1/00, C07J 21/00, C07J 41/00, C07J 43/00, C07J 51/00, C07J 71/00, A61K 31/567, A61K 31/58, A61P 5/36

(54) **PROGESTERONE ANTAGONISTS**
PROGESTERONANTAGONISTEN
ANTAGONISTES DE PROGESTÉRONE

(30) Priority: 28.07.2011 US 201113193426
(43) Date of publication of application: 04.06.2014
(73) Proprietor: Evestra, Inc., San Antonio, Texas 78227-5301 (US)
(72) Inventor: NICKISCH, Klaus, 12307 Berlin (DE); NARKUNAN, Kesavaram, San Antonio, Texas 78249 (US); DEBNATH, Baishakhi, San Antonio, Texas 78251 (US); SANTHAMMA, Bindu, San Antonio, Texas 78253 (US)
(74) Representative: AWA Sweden AB
(86) International application number: PCT/US2012/048805
(87) International publication number: WO 2013/016725

(56) References cited:
- EP-A2- 0 057 115
- WO-A1-00/34306
- WO-A1-98/34947
- WO-A1-03/093292
- WO-A1-2008/058767
- US-A1- 2002 143 000

## Description

### BACKGROUND OF THE INVENTION

### 1. Field of the Invention

The present invention relates to the identification of a class of compounds that behave either as pure antiprogestins or as antiprogestins with partial agonistic activity, also called mesoprogestins. Pure antiprogestins has been known to suppress the growth of cancer and other proliferative diseases, whereas mesoprogestins has been shown to be useful in the treatment of fibroids and endometriosis etc. The present invention also relates to processes of preparation and the use in therapy of such novel compounds.

### 2. Description of the Relevant Art

In the past, progesterone antagonists have been postulated to be of potential benefit in the treatment of breast cancer where the primary lesion contains both estrogen and progesterone receptors. In a recent study of an *in vivo* rat model of progesterone receptor positive breast cancer, it was shown that the administration of a new antiprogestin (Proellex, CDB-4124) resulted in a regression of tumor size as well as a decrease in the development of new tumors. FIG. 1 shows a series of selected progesterone receptor modulators that have been shown to be effective *in vitro* and *in vivo.* The prototype antagonist, Mifepristone (see FIG. 1), is characterized by the 19-nor-4,9-diene steroid nucleus, the 17α-propynyl-17β-hydroxy functionality, and the 11β-(4-dimethylamino)phenyl functional group which is believed to be responsible for its antagonistic activity. While Mifepristone is a potent progesterone antagonist, its long-term clinical use is limited due to its overt glucocorticoid receptor antagonism. Subsequent development undertaken by several groups has led to the discovery of several novel progesterone antagonists that are both more active than Mifepristone and more dissociated in relation to glucocorticoid antagonism. Some notable examples as outlined above in FIG. 1 and include Onapristone, Asoprisnil, ORG-33628, Proellex, and Lonaprisan (ZK-230211). 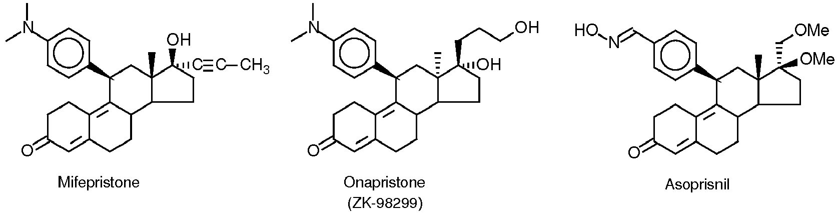 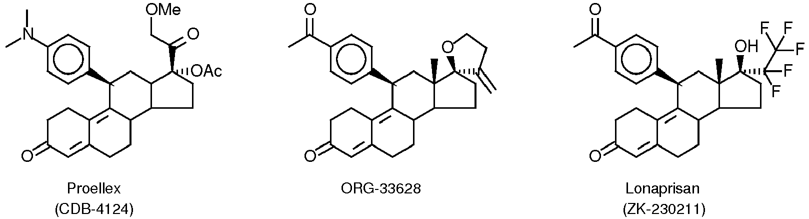 FIG. 1 depicts several known progesterone receptor modulators and pure antagonists.

Of these examples, Lonaprisan is most notable in that it exhibits high antiprogestagenic activity and displays only marginal antiglucocorticoid effects.

US-A-2002/143000 discloses a method of inhibiting the occurrence of advanced endometrium in a female mammal undergoing fertility treatment, wherein a 17α-fluoralkylated progesterone receptor antagonist, e.g. Lonapristone (ZK-230211) and the corresponding 11β-(4'-(3-pyridyl)-phenyl]-derivative (anti-progestin) is involved.

WO-A-00/34306 discloses potential compounds that can be either antagonists or selective progesterone receptor modulators, e.g. the 17α-C≡C-CF₃ analogue of Lonapristone (anti-progestin).

While antiprogestin therapies have been effective in the treatment of some forms of cancer (including breast cancers), there is still a need to develop more effective therapies.

### SUMMARY OF THE INVENTION

The present invention refers to a compound having the structure according to claim 1, and to a compound having the structure according to claim 2 for use in the treatment of cancer in a subject.

### BRIEF DESCRIPTION OF THE DRAWINGS

Advantages of the present invention will become apparent to those skilled in the art with the benefit of the following detailed description of embodiments and upon reference to the accompanying drawings in which:
While the invention may be susceptible to various modifications and alternative forms, specific embodiments thereof are shown by way of example in the drawings and will herein be described in detail. The drawings may not be to scale.

### DETAILED DESCRIPTION OF THE PREFERRED EMBODIMENTS

As used in this specification and the appended claims, the singular forms "a", "an", and "the" include singular and plural referents unless the content clearly dictates otherwise. Thus, for example, reference to "a linker" includes one or more linkers.

Unless defined otherwise, all technical and scientific terms used herein have the same meaning as commonly understood by one of ordinary skill in the art.

Compounds described herein embrace both racemic and optically active compounds. Chemical structures depicted herein that do not designate specific stereochemistry are intended to embrace all possible stereochemistries.

It will be appreciated by those skilled in the art that compounds having one or more chiral center(s) may exist in and be isolated in optically active and racemic forms. Some compounds may exhibit polymorphism. It is to be understood that the present invention encompasses any polymorphic form of a compound. As used herein, the term "single stereoisomer" refers to a compound having one or more chiral center that, while it can exist as two or more stereoisomers, is isolated in greater than about 95% excess of one of the possible stereoisomers. As used herein a compound that has one or more chiral centers is considered to be "optically active" when isolated or used as a single stereoisomer.

The term "alkyl" as used herein generally refers to a chemical substituent containing the monovalent group CₙH₂ₙ, where n is an integer greater than zero. In some embodiments n is 1 to 12. The term "alkyl" includes a branched or unbranched monovalent hydrocarbon radical. Examples of alkyl radicals include, but are not limited to: methyl, ethyl, propyl, isopropyl, butyl, iso-butyl, sec-butyl, pentyl, 3-pentyl, hexyl, heptyl, octyl, nonyl, decyl, undecyl, dodecyl. When the alkyl group has from 1-6 carbon atoms, it is referred to as a "lower alkyl." Suitable lower alkyl radicals include, but are not limited to, methyl, ethyl, n-propyl, i-propyl, 2-propenyl (or allyl), n-butyl, t-butyl, and i-butyl (or 2-methylpropyl).

The term "substituted alkyls" as used herein generally refers to alkyl radicals that include one or more functional groups attached to any carbon of the alkyl radical. Functional groups include, but are not limited to, aryl, aralkyl, acyl, halogens, hydroxyl, amino, alkylamino, acylamino, acyloxy, alkoxy, and mercapto. As used herein the term "substituted lower alky" refers to an alkyl residue having from 1-6 carbon atoms and one or more functional groups attached to any carbon of the alkyl radical.

The term "alkoxy" generally refers to an -OR group, where R is a lower alkyl, substituted lower alkyl, aryl, substituted aryl, aralkyl or substituted aralkyl. Suitable alkoxy radicals include, but are not limited to, methoxy, ethoxy, phenoxy, t-butoxy, methoxyethoxy, and methoxymethoxy.

The term "acyloxy" is used herein to refer to an organic radical derived from an organic acid by the removal of a hydrogen. The organic radical can be further substituted with one or more functional groups including, but not limited to, alkyl, aryl, aralkyl, acyl, halogen, amino, thiol, hydroxyl, alkoxy. etc. Suitable acyloxy groups include, for example, acetoxy, i.e., CH₃COO-, which is derived from acetic acid.

The term "halogen" is used herein to refer to fluorine, bromine, chlorine and iodine atoms.

The term "hydroxyl" is used herein to refer to the group -OH.

The term "alkylacyl" denotes groups -C(O)R where R is alkyl or substituted alkyl, aryl or substituted aryl as defined herein.

The term "cycloalkylacyl" denotes groups -C(O)R where R is a cycloalkyl or substituted cycloalkyl such as, for example, cyclopropylacyl-, cyclopentylacyl and cyclohexylacyl.

The term "aryl" is used to refer to an aromatic substituent which may be a single ring or multiple rings which are fused together, linked covalently, or linked to a common group such as an ethylene moiety. Aromatic ring(s) include but are not limited to phenyl, naphthyl, biphenyl, diphenylmethyl, and 2,2-diphenyl-1-ethyl. The aryl group may also be substituted with substituents including, but not limited to, alkyl groups, halogen atoms, nitro groups, carboxyl groups, alkoxy, and phenoxy to give a "substituted aryl group." Substituents may be attached at any position on the aryl radical which would otherwise be occupied by a hydrogen atom.

The term "heterocycle" as used herein generally refers to a closed-ring structure, in which one or more of the atoms in the ring is an element other than carbon. Heterocycle may include aromatic compounds or non-aromatic compounds. Heterocycles may include rings such as thiophene, pyridine, isoxazole, phthalimide, pyrazole, indole, furan, or benzo-fused analogs of these rings. Examples of heterocycles include tetrahydrofuran, morpholine, piperidine, pyrrolidine, and others. In some embodiments, "heterocycle" is intended to mean a stable 5- to 7-membered monocyclic or bicyclic or 7- to 10-membered bicyclic heterocyclic ring which is either saturated or unsaturated, and which consists of carbon atoms and from 1 to 4 heteroatoms (e.g., N, O, and S) and wherein the nitrogen and sulfur heteroatoms may optionally be oxidized, and the nitrogen may optionally be quaternized, and including any bicyclic group in which any of the above-defined heterocyclic rings is fused to a benzene ring. In some embodiments, heterocycles may include cyclic rings including boron atoms. The heterocyclic ring may be attached to its pendant group at any heteroatom or carbon atom that results in a stable structure. The heterocyclic rings described herein may be substituted on carbon or on a nitrogen atom if the resulting compound is stable. Examples of such heterocycles include, but are not limited to, 1H-indazole, 2-pyrrolidonyl, 2H,6H-1,5,2-dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzofuranyl, benzothiophenyl, carbazole, chromanyl, chromenyl, cinnolinyl, decahydroquinolinyl, furanyl, furazanyl, imidazolidinyl, imidazolinyl, imidazolyl, indolinyl, indolizinyl, indolyl, isobenzofuranyl, isochromanyl, isoindolinyl, isoindolyl, isoquinolinyl (benzimidazolyl), isothiazolyl, isoxazolyl, morpholinyl, naphthyridinyl, octahydroisoquinolinyl, oxazolidinyl, oxazolyl, phenanthridinyl, phenanthrolinyl, phenarsazinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridinyl, pyridyl, pyrimidinyl, pyrrolidinyl, pyrrolinyl, pyrrolyl, quinazolinyl, quinolinyl, quinoxalinyl, quinuclidinyl, carbolinyl, tetrahydrofuranyl, tetrahydroisoquinolinyl, tetrahydroquinolinyl, tetrazolyl, thianthrenyl, thiazolyl, thienyl, thiophenyl, triazinyl, xanthenyl. Also included are fused ring and spiro compounds containing, for example, the above heterocycles.

The term "alkyl carbonate" is used herein to refer to the group -OC(O)OR, where R is alkyl, substituted alkyl, aryl, or substituted aryl as defined herein.

The term "S-alkyl" is used herein to refer to the group -SR, where R is lower alkyl or substituted lower alkyl.

The term "S-acyl" is used herein to refer to a thioester derived from the reaction of a thiol group with an acylating agent. Examples of S-acyl radicals include, but are not limited to, S-acetyl, S-propionyl and S-pivaloyl. Those of skill in the art will know that S-acyl refers to such thioesters regardless of their method of preparation.

The terms "N-oxime" and "N-alkyloxime" are used herein to refer to the group =N-OR⁵, where R⁵ is for example, hydrogen (N-oxime) or alkyl (N-alkyloxime). Those of skill in the art will recognize that the oximes may include the syn-isomer, the anti-isomer, or a mixture of both the syn- and anti-isomers.

As used herein the terms "alkenyl" and "olefin" generally refer to any structure or moiety having the unsaturation C=C. Examples of alkenyl radicals include, but are not limited to vinyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 1-nonenyl, 2-nonenyl, 3-nonenyl, 4-nonenyl, 5-nonenyl, 6-nonenyl, 7-nonenyl, 8-nonenyl, 1-decenyl, 2-decenyl, 3-decenyl, 4-decenyl, 5-decenyl, 6-decenyl, 7-decenyl, 8-decenyl, 9-decenyl; 1-undecenyl, 2-undecenyl, 3-undecenyl, 4-undecenyl, 5-undecenyl, 6-undecenyl, 7-undecenyl, 8-undecenyl, 9-undecenyl, 10-undecenyl, 1-dodecenyl, 2-dodecenyl, 3-dodecenyl, 4-dodecenyl, 5-dodecenyl, 6-dodecenyl, 7-dodecenyl, 8-dodecenyl, 9-dodecenyl, 10-dodecenyl, and 11-dodecenyl.

The term "fluorinated alkenyl" as used herein generally refers to alkenyl radicals that include one or more fluorine atoms attached to any carbon of the alkenyl radical in place of a hydrogen atom.

As used herein, the term "alkynyl" generally refers to any structure or moiety having the unsaturation C=C. Examples of alkynyl radicals include, but are not limited to: ethynyl, 1-propynyl, 2-propynyl, 1-butynyl, 2-butynyl, and 3-butynyl.

The term "fluorinated alkynyl" as used herein generally refers to alkynyl radicals that include one or more fluorine atoms attached to any carbon of the alkynyl radical in place of a hydrogen atom.

The term "pharmaceutically acceptable salts" includes salts prepared from by reacting pharmaceutically acceptable non-toxic bases or acids, including inorganic or organic bases, with inorganic or organic acids. Pharmaceutically acceptable salts may include salts derived from inorganic bases include aluminum, ammonium, calcium, copper, ferric, ferrous, lithium, magnesium, manganic salts, manganous, potassium, sodium, zinc, etc. Examples include the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-dibenzylethylenediamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, etc.

### Progesterone Antagonists

A progesterone antagonist, as described but not claimed herein, may have the structure of formula (**I**): 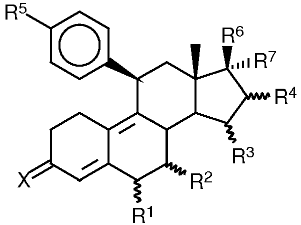 In which
R¹ is a hydrogen atom, a straight-chain C₁-C₅ alkyl group, a branched C₁-C₅ alkyl group, a C₃-C₅ cycloalkyl group or a halogen atom;
R² is a hydrogen atom, a straight-chain C₁-C₅ alkyl group, a branched C₁-C₅ alkyl group, a C₃-C₅ cycloalkyl group or a halogen atom; or
R¹ and R² together are a methylene group,
R³ is a hydrogen atom, a straight-chain C₁-C₅ alkyl group, a branched C₁-C₅ alkyl group, a C₃-C₅ cycloalkyl group or a halogen atom;
R⁴ is a hydrogen atom, a straight-chain C₁-C₅ alkyl group, a branched C₁-C₅ alkyl group, a C₃-C₅ cycloalkyl group or a halogen atom; or
R³ and R⁴ together are an additional bond or a methylene group,
R⁵ is a radical Y or an aryl radical that is optionally substituted with Y,
Y is a hydrogen atom, a halogen atom, -OR⁸, -NO₂, -N₃, -CN, -NR^{8a}R^{8b}, -NHSO₂R⁸, -CO₂R⁸, C₁-C₁₀ alkyl, C₁-C₁₀ substituted alkyl, C₁-C₁₀ cycloalkyl, C₁-C₁₀ alkenyl, C₁-C₁₀ alkynyl, C₁-C₁₀ alkoxy, C₁-C₁₀ cycloalkoxy, C₁-C₁₀ alkanoyloxy, benzoyloxy, arylacyl, C₁-C₁₀-alkylacyl, C₁-C₁₀-cycloalkylacyl, C₁-C₁₀ hydroxyalkyl, aryl arylalkyl, heteroaryl with two or three heteroatoms, or heteroarylacyl containing up to three heteroatoms;
R⁶ is -OH, -OR⁸, -OC(O)R⁸, -C≡C-R¹⁰, -C(O)CH₂R⁸, alkyl, -H, -(CH₂)ₘCH₂R⁹, or -CH=CH-(CH₂)ₘ-R⁹;
R⁷ is -OH, -OR⁸, -OC(O)R⁸, -C≡C-R¹⁰, -C(O)CH₂R⁸, alkyl, -H, -(CH₂)ₘCH₂R⁹, -CH=CH-(CH₂)ₘ-R⁹; a radical of formula CₙFₘHₒ where n is 2,3,4,5 or 6 with m≥1 and m+o<2n+1, or -CF₂-CH₂-CH₃; or
R⁶ and R⁷ together form 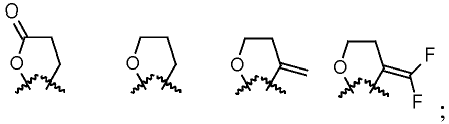
R⁸ is H, alkyl, alkyloxy, or aryl;
R⁹ is H, cyano, hydroxyl, alkoxy, acyloxy; and
R¹⁰ is H, chloro, fluoro, alkyl, hydroxyalkyl;
wherein the wavy lines represent a substituent in either the α- or β- position.

In a described but unclaimed embodiment:
R⁵ is a radical Y or an aryl radical that is optionally substituted with Y, whereby Y is a hydrogen atom, a halogen atom, -OR⁸, -NO₂, -N₃, -CN, -NR^{8a}R^{8b}, -NHSO₂R⁸, -CO₂R⁸, C₁-C₁₀ alkyl, C₁-C₁₀ substituted alkyl, C₁-C₁₀ cycloalkyl, C₁-C₁₀ alkenyl, C₁-C₁₀ alkynyl, C₁-C₁₀ alkoxy, C₁-C₁₀ cycloalkoxy, C₁-C₁₀ alkanoyloxy, benzoyloxy, aryl acyl, C₁-C₁₀-alkylacyl, C₁-C₁₀-cycloalkylacyl, C₁-C₁₀ hydroxyalkyl, aryl or arylalkyl, a five or six membered heterocyclic radical containing up to three heteroatoms,
R⁶ is -OH, -OR⁸, or -OC(O)R⁸; and
R⁷ stands for a radical of formula CₙFₘHₒ whereby n is 2,3,4,5 or 6 with m≥1 and m+o<2n+1 or R⁶ = OH and R⁷ = -CF₂-CH₂-CH₃
In some specific embodiments, R⁷ is -C≡C-CF₃, -C=CH-CF₃, -CH₂-CF=CF₂, -CH₂-CF₂-CH=CH₂, -CF₂-CH₂-CH₃, or -C₂F₅.
In an embodiment:
R⁵ is cycloalkylacyl or arylacyl or heteroaryl with two or three heteroatoms or heteroarylacyl containing upto three heteroatoms; and
R⁶ and R⁷ are:
   -OR⁸ and -≡-R¹⁰ respectively;
   -≡-R¹⁰ and -OR⁸ respectively;
   -OR⁸ and -COCH₂R⁸ respectively;
   -COCH₂R⁸ and -OR⁸ respectively;
   -CH₃ and -COCH₂R⁸ respectively;
   -COCH₂R⁸ and -CH₃ respectively
   -H and -COCH₂R⁸ respectively;
   -COCH₂R⁸ and -H respectively;
   -OR⁸ and -(CH₂)ₘCH₂-R⁹ respectively;
   -OR⁸ and -CH=CH-(CH₂)ₘ-R⁹ respectively; or
R⁶ and R⁷ combine to form 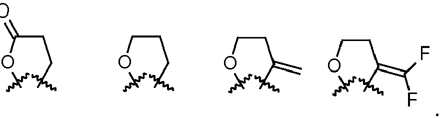
R⁷ stands for a radical of formula CₙFₘHₒ whereby n is 2,3,4,5 or 6 with m = 0,1,2,3 and m+o≤2n+1.
In some specific embodiments, R⁷ is -C≡C-CH₃, -CH₂-CH₂-CH₂-OH, or -CH=CH-CH₂-OH.

The wavy lines in the embodiments described herein represent that the substituent in question can be in α- or β-position.

As noted above, the introduction of a fluorinated substituent, as, for example, in Lonaprisan had a significant effect on the antiprogestagenic activity of the compound. The introduction of unsaturated fluorinated groups at the C-17α-position of 11β-aryl-19-nor steroids, in one embodiment, may produce compounds with higher antiprogestational activity and reduced activity towards other steroid receptors than known progesterone antagonists.

In one, described but unclaimed a progesterone antagonist may have the structure of formula (**I**): 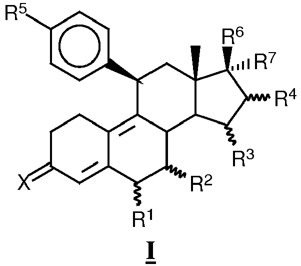 In which
R¹ is a hydrogen atom, a straight-chain C₁-C₅ alkyl group, a branched C₁-C₅ alkyl group, a C₃-C₅ cycloalkyl group or a halogen atom;
R² is a hydrogen atom, a straight-chain C₁-C₅ alkyl group, a branched C₁-C₅ alkyl group, a C₃-C₅ cycloalkyl group or a halogen atom; or
R¹ and R² together are a methylene group,
R³ is a hydrogen atom, a straight-chain C₁-C₅ alkyl group, a branched C₁-C₅ alkyl group, a C₃-C₅ cycloalkyl group or a halogen atom;
R⁴ is a hydrogen atom, a straight-chain C₁-C₅ alkyl group, a branched C₁-C₅ alkyl group, a C₃-C₅ cycloalkyl group or a halogen atom; or
R³ and R⁴ together are an additional bond or a methylene group,
R⁵ is a radical Y or an aryl radical that is optionally substituted with Y, whereby Y is a hydrogen atom, a halogen atom, -OR⁸, -NO₂, -N₃, -CN, -NR^{8a}R^{8b}, -NHSO₂R⁸, -CO₂R⁸, C₁-C₁₀ alkyl, C₁-C₁₀ substituted alkyl, C₁-C₁₀ cycloalkyl, C₁-C₁₀ alkenyl, C₁-C₁₀ alkynyl, C₁-C₁₀ alkoxy, C₁-C₁₀ cycloalkoxy, C₁-C₁₀ alkanoyloxy, benzoyloxy, aryl acyl, C₁-C₁₀-alkylacyl, C₁-C₁₀-cycloalkylacyl, C₁-C₁₀ hydroxyalkyl, aryl or arylalkyl, a five or six membered heterocyclic radical containing up to three heteroatoms,
R⁶ stands for a free, etherified or esterified hydroxyl group,
R⁷ stands for a radical of formula CₙFₘHₒ whereby n is 2,3,4,5 or 6 with m≥1 and m+o<2n+1 or R⁶ = OH and R⁷ = -CF₂-CH₂-CH₃
R⁸ = hydrogen atom, hydroxyl group, O-alkyl, O-alkyloxy

According to another described but unclaimed a progesterone antagonist may have the structure of formula (**I**):
Wherein
R¹ is a hydrogen atom, a straight-chain C₁-C₅ alkyl group, a branched C₁-C₅ alkyl group, a C₃-C₅ cycloalkyl group or a halogen atom;
R² is a hydrogen atom, a straight-chain C₁-C₅ alkyl group, a branched C₁-C₅ alkyl group, a C₃-C₅ cycloalkyl group or a halogen atom; or
R¹ and R² together are a methylene group,
R³ is a hydrogen atom, a straight-chain C₁-C₅ alkyl group, a branched C₁-C₅ alkyl group, a C₃-C₅ cycloalkyl group or a halogen atom;
R⁴ is a hydrogen atom, a straight-chain C₁-C₅ alkyl group, a branched C₁-C₅ alkyl group, a C₃-C₅ cycloalkyl group or a halogen atom; or
R³ and R⁴ together are an additional bond or a methylene group and if
R⁵ is cycloalkylacyl or arylacyl or heteroaryl with two or three heteroatoms or heteroarylacyl containing upto three heteroatoms then
R⁶ and R⁷ are
   -OR⁸ / -≡-Y
   -≡-Y / -OR⁸
   -OR⁸ / -COCH₂R⁸
   -COCH₂R⁸ / -OR⁸
   -CH₃ / -COCH₂R⁸
   -COCH₂R⁸ / -CH₃
   -H / -COCH₂R⁸
   -COCH₂R⁸ / -H
   -OR⁸ / -(CH₂)ₘCH₂-R⁹
   -OR⁸ / -CH=CH-(CH₂)ₘ-R⁹ 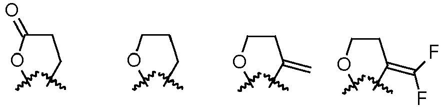
Y = H, chloro, fluoro, alkyl, hydroxyalkyl
R⁸ = hydrogen atom, hydroxyl group, O-alkyl, O-alkyloxy
R⁹ = hydrogen, cyano, hydroxyl, alkoxy, acyloxy
R7 stands for a radical of formula CₙFₘHₒ whereby n is 1,2,3,4,5 or 6 with m = 0,1,2,3 and m+o≤2n+1

The wavy lines represent that the substituent in question can be in α- or β-position.

A specific example of a compound having the formula (I) include a compound according to the present invention having the structure (**1**): 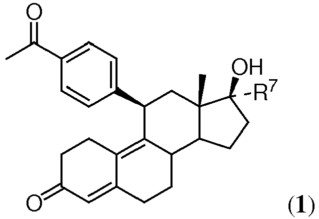 wherein R⁷ is
**1a:** -C≡C-CF₃,
**1b:** (E)-CH=CH-CF₃,
**1c:** -CH₂-CF=CF₂,
**Id:** -CF₂-CH=CH₂, or
**1e:** -CF₂-CH₂-CH₃, wherein 1a and 1e do not belong to the present invention.

Another example of a compound having the formula (**I**) include a compound having the structure (**2**): 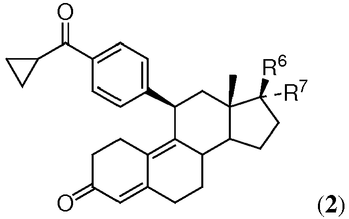 Wherein:
**2a:** R⁶ is -OH and R⁷ is C₂F₅,
**2b:** R⁶ is -OH and R⁷ is -C≡C-CH₃,
**2c:** R⁶ is -OH and R⁷ is -CH₂-CH₂-CH₂-OH,
**2d:** R⁶ is -OH and R⁷ is (Z)-CH=CH-CH₂-OH; or
**2e:** R⁶ and R⁷ together form

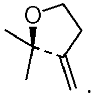

Another example of a compound having the formula (**I**) include a compound according to the present invention having the structure (**3**): 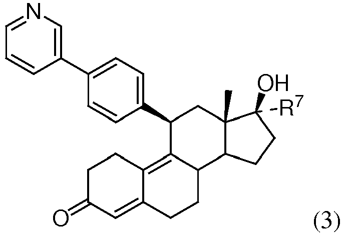 wherein R⁷ is :
**3a**: (E)-CH=CH-CF₃ or
**3b**: -CF₂-CH=CH₂.

Another example of a compond having the formula (**I**) include a compond having the structure (**4**)

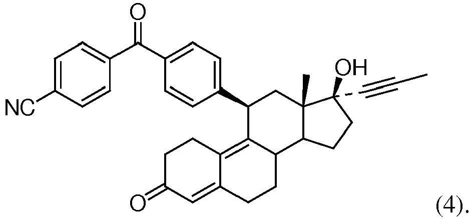

Synthesis of compounds **1a, 1b, 1c, 1d, 1e, 2a, 2b, 2c, 2d, 2e, 3a, 3b** and **4** may be prepared according to the following schemes.

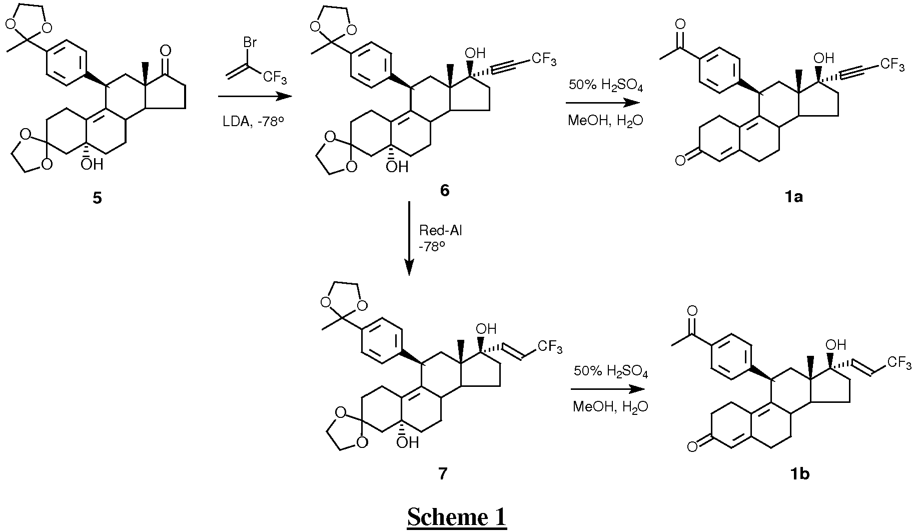

The intermediate **5** may be synthesized following the procedure of Rao et al., Steroids, 1998, 63, 523. Treatment of 2-bromo-3,3,3-trifluoropropene with 2 eqts of LDA generated the required 3,3,3-trifluoropropynyllithium at -78° C and was added to **5** to obtain **6.** Red-Al reduction of **6** at -78° C yielded selectively the trans-double bond. Both **6** and **7** upon hydrolysis yielded **1a** and **1b** respectively.

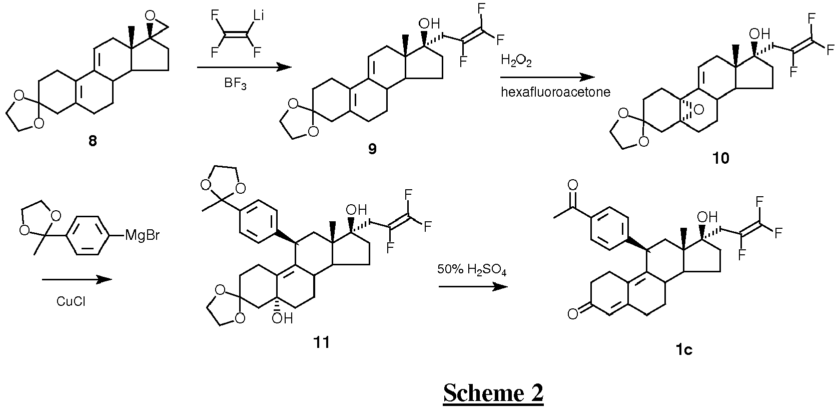

Opening of epoxide **8** with trifluorovinyllithium (generated from 1,1,1,2-tetrafluoroethane and n-BuLi at -78° C) in presence of boron trifluoride etherate afforded **9.** Subsequent epoxidation, conjugate Grignard addition and hydrolysis yielded **1c.**

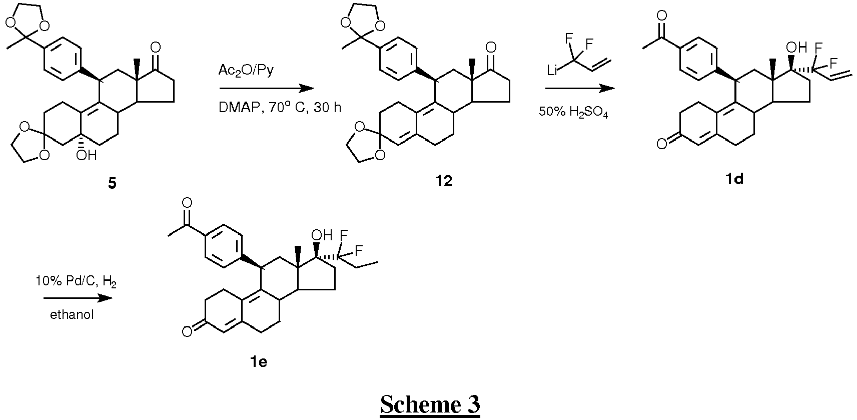

Dehydration of 17-keto derivative (**5**) was achieved by treating with excess of acetic anhydride in pyridine at 70° C for 30h to afford **12.** Addition of difluoroallyllithium to **12** at -100° C generated the addition product, which upon acid hydrolysis yielded **1d.** The side chain double bond at C-17 can be selectively hydrogenated using 10% Pd/C under hydrogen atmosphere to provide **1e.**

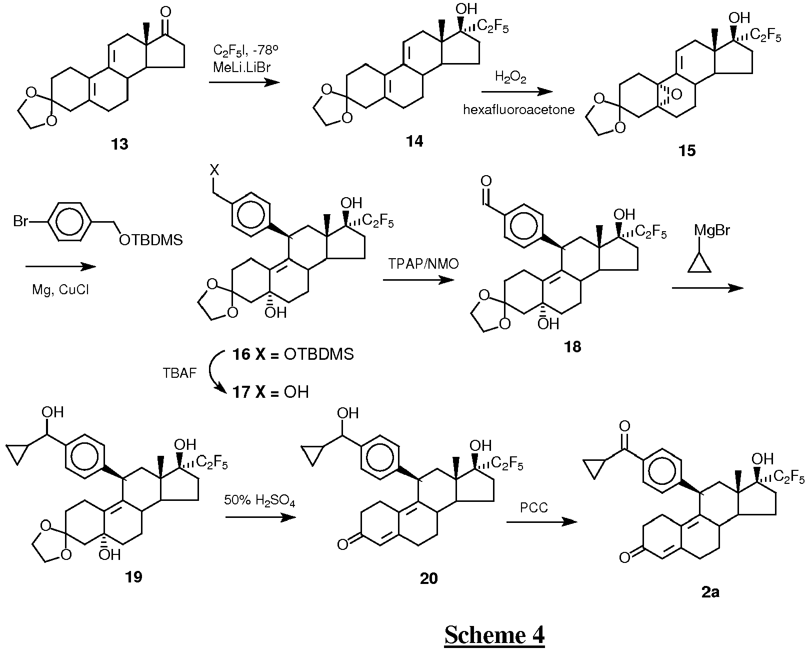

Addition of pentafluoroethyllithium to **13** was followed as described in Fuhrmann Ulrike et al., WO2008058767 to afford **14.** Subsequent epoxidation and aryl Grignard addition yielded **16.** Deprotection of silyl protection using TBAF, followed by oxidation in the presence of TPAP/NMO resulted in the required benzaldehyde **18.** Addition of cyclopropyl magnesium bromide, acid hydrolysis followed by oxidation obtained the final product **2a.**

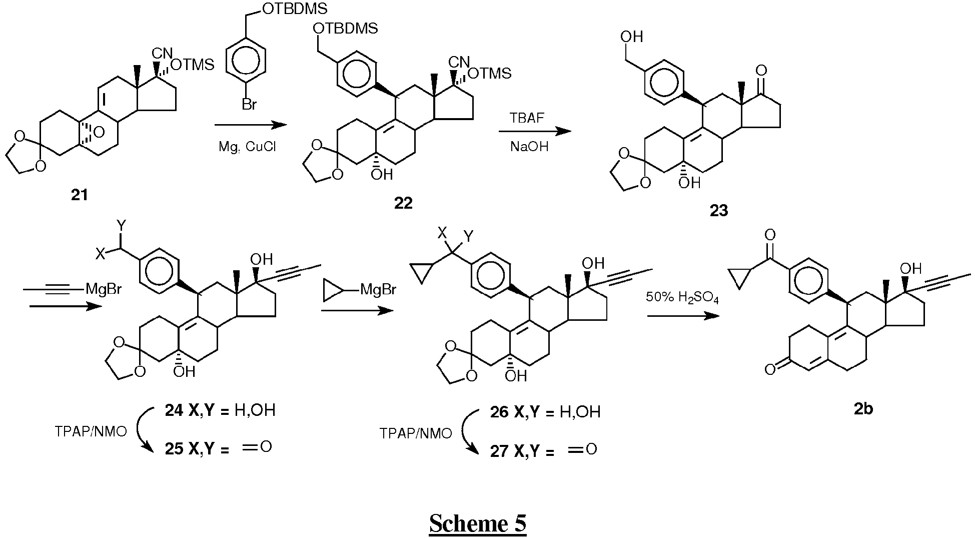

Addtion of aryl cuprate to epoxide **21,** followed by desilylation transformed to diol **23.** Treatment of **23** with excess of propynyl magnesium bromide introduced propynyl group at C-17. Oxidation of benzylic alcohol **24,** followed by addition of cyclopropyl magnesium bromide afforded **26,** which upon further oxidation and acid hydrolysis lead to the required product **2b.**

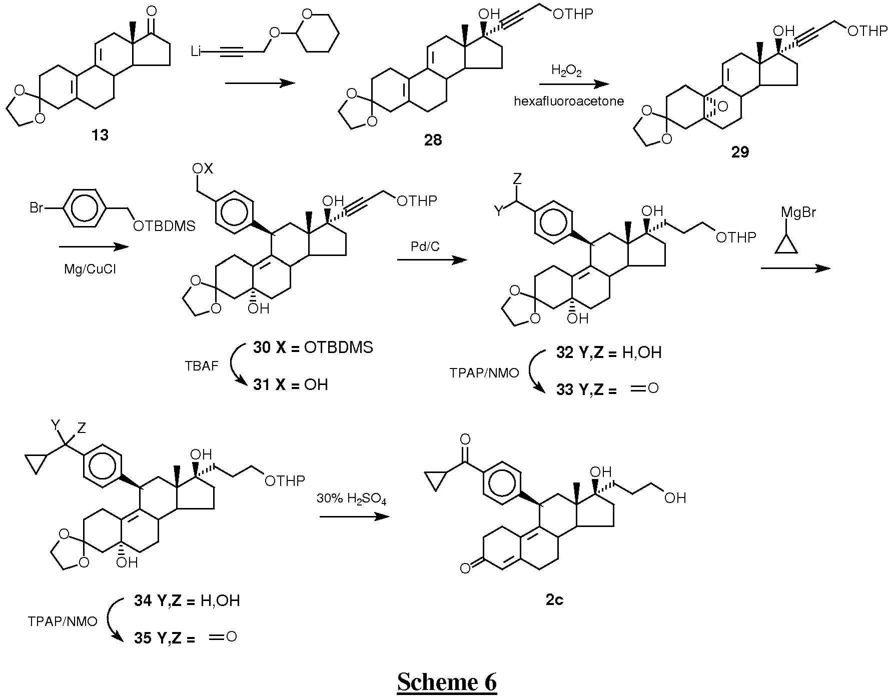

Addition of excess of lithiated tetrahydropyran protected propargyl alcohol to **13** led to **28,** which upon epoxidation followed by conjugate aryl Grignard addition resulted in **30.** Desilylation of **30** with TBAF, palladium/carbon mediated hydrogenation and an oxidation afforded the corresponding benzaldehyde (**33**). Final steps involving addition of cyclopropyl Grignard, oxidation and an acid mediated hydrolysis led to the final product **2c.**

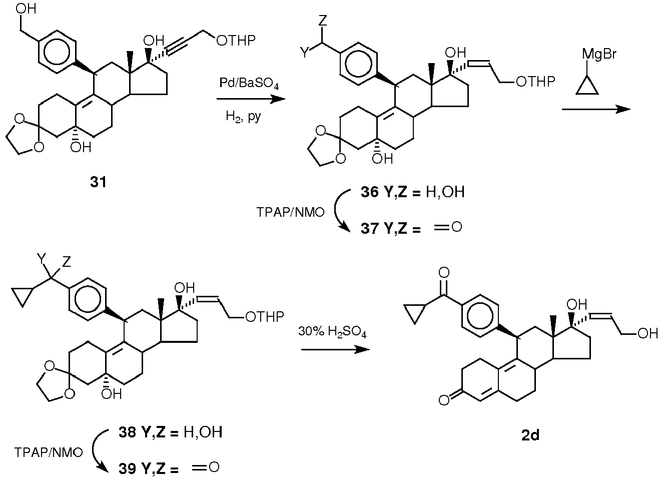

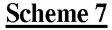

Scheme 7 followed the similar transformation as in scheme 6, except the hydrogenation was carried out using Pd/BaSO₄ to obtain the corresponding cis-olefin. Remaining steps of oxidation, cyclopropyl addition, oxidation and hydrolysis resulted in **2d.**

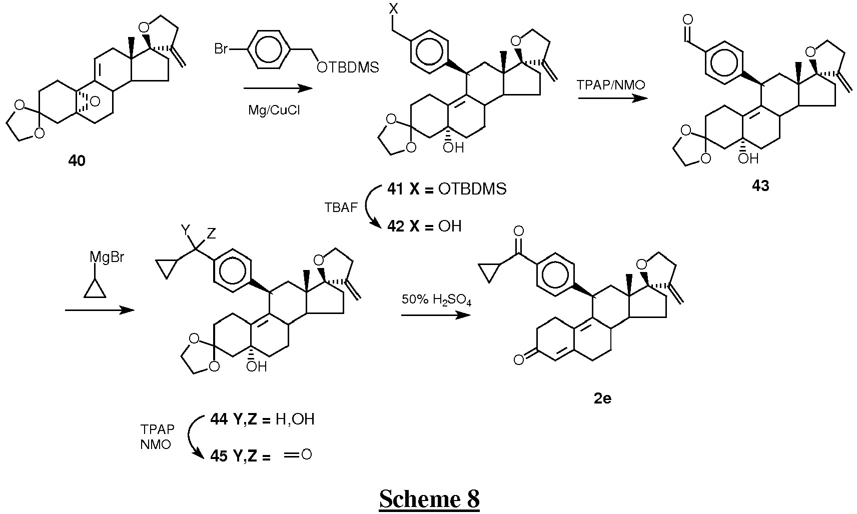

Epoxide **40** can be prepared according to Jiang et al. "New progesterone receptor antagonists: Phosphorus-containing 11β-aryl-substituted steroids." Bioorg Med Chem (2006) 14:6726-6732. Addition of aryl cuprate followed by deprotection afforded **42,** which upon oxidation and cyclopropyl Grignard addition led to the diol **44.** Oxidation of **44** and acid hydrolysis gave the required **2e.**

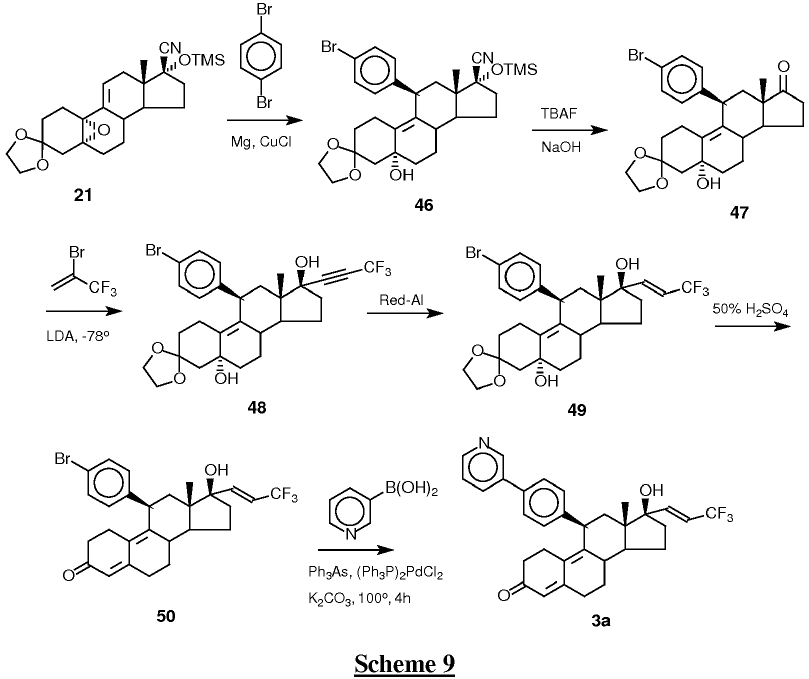

Epoxide **21** was treated with excess of p-bromophenyl magnesium bromide in presence of catalytic copper chloride to yield **46,** which upon desilylation led to 17-keto derivative **47.** Addition of excess 3,3,3-trifluorpropinyllithium to **47,** followed by Red-Al reduction yielded **49.** Hydrolysis of **49** followed by a palladium mediated Suzuki coupling gave **3a.**

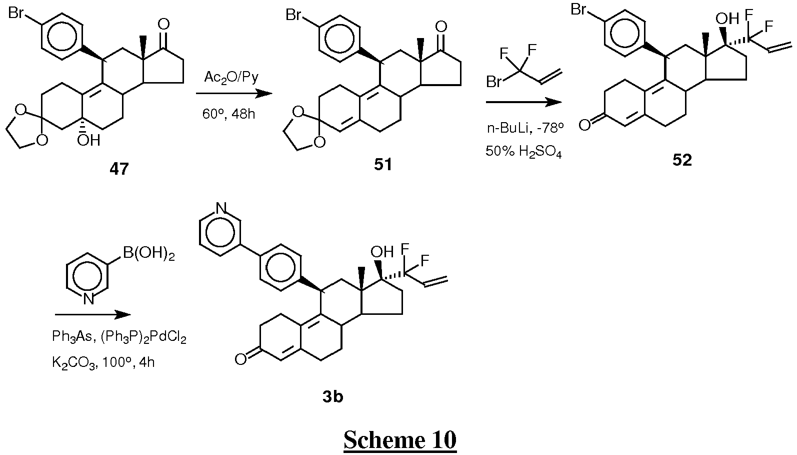

Dehydration of 17-keto derivative **47** was achieved by treating with excess of acetic anhydride in pyridine at 60° C for 48h to afford **51.** Addition of difluoroallyl lithium to **51** at -100° C generated the addition product, which upon acid hydrolysis yielded **52.** Final palladium mediated Suzuki coupling of **52** with pyridinyl-3-boronic acid yielded **3b.**

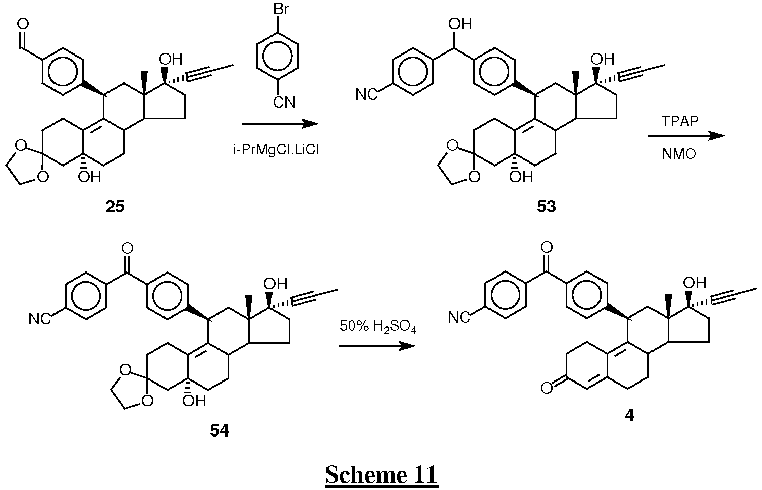

Addition of p-cyanophenyl magnesium bromide to the aldehyde **25** gave the addition product **53,** which upon oxidation followed by hydrolysis yielded **4.**

Any suitable route of administration may be employed for providing a patient with an effective dosage of the progesterone antagonist compounds described herein. For example, oral, rectal, topical, parenteral, ocular, pulmonary, nasal, and the like may be employed. Dosage forms include tablets, troches, dispersions, suspensions, solutions, capsules, creams, ointments, aerosols, and the like. In certain embodiments, it may be advantageous that the compositions described herein be administered orally.

The agonist and antagonist actions of the described progesterone antagonists may be tested using breast cancer cells. Established human breast cancer cell lines, such as MCF-7, T47D, MDMB231 and SKBR-3 and derivatives of these cell lines with/without PR expression, may be used to test the effect of the novel progesterone antagonists.

In one embodiment, a progesterone receptor reporter gene system may be used to evaluate the agonist and antagonist activities of the subject progesterone antagonists. Agonist and antagonist activities may further verified using a progesterone transactivation assay along with a proliferative effect assay of progesterone antagonists on these cells.

The in vitro biological activity of the progesterone antagonists may be compared to the activity of controls P4 (agonist) and RU486 (antagonist) using a cell based progesterone receptor element (PRE)-luciferase assay, as described in Giangrande et al. "The Opposing Transcriptional Activities of the Two Isoforms of the Human Progesterone Receptor Are Due to Differential Cofactor Binding." Mol Cell Biol (2000) 20:3102-3115, which is incorporated herein by reference. The luciferase (luc) reporters 2XPRE-tk-luc contain two copies of the progesterone response element (PRE) upstream of a thymidine kinase (tk) promoter. This vector has been used in numerous studies to test the effect of various progesterone antagonists. Test progesterone antagonists may be evaluated for PR agonism and antagonism in this assay. Testing the PR agonist and antagonist activities with different concentrations of the progesterone antagonists may be used to determine their ability to block or enhance the PRE-luciferase activity and as a measure of their ability to bind and influence PR regulation using the T47D breast cancer cell line. This cell line expresses both human PR-A and PR-B forms of PR and is widely used for testing P4/PR effects. After determining the optimum concentration, the progesterone antagonists may be tested in different cell lines at the optimum concentration. Also their agonism and antagonism may be tested in PR-dependent transactivation assay.

In the luciferase assay, the reporter vector is first transfected into cells. After a limited amount of time, the cells are lysed and the substrate of luciferase, luciferin, is introduced into the cellular extract along with Mg and excess ATP. Under these conditions, luciferase enzyme expressed by the reporter vector will catalyze the oxidative carboxylation of luciferin. The luminescence from this chemical reaction can be read and quantified by a luminometer. The amount of light detected from the cell lysate correlates directly with the binding activity of the transcription factor. The Empty Control Vector may be used as a negative control for subtracting any background. The Empty Control Vector does not contain the transcription factor response element insert; it only contains the minimal TATA promoter and does not respond to any specific transactivation compound.

Transfections may be performed in 80% confluent 24-h-old cultures. For transient transfection, 200mg/well of PRE-luciferase DNA may be used. Lipofectamine 2000 may be used for transfection following the manufacturer's instructions. Unless otherwise specified, 5 ng/well of other optical reporters may be used for transfection normalization in the transient transfection studies. The cells may be assayed after 24h incubation at 37°C at 5% CO₂ with a specific concentration for each progesterone antagonist. The transfected cells may be lysed in 200 ml of ice-cold 1X passive lysis buffer supplied by Promega and may then be shaken for 15 min on ice. The cell lysates may be centrifuged for 5 min at 1.3 x 10⁴ g at 4°C to remove cell debris. To determine Renilla luciferase activity, 20 ml of supernatant may be assayed by addition of 0.5 mg of coelenterazine in 100 ml of 0.5M sodium PBS at pH 7.0 (PBS), followed by photon counting in the luminometer (model T 20/20; Turner Designed, Sunnyvale, CA) for 10 sec. Firefly luciferase activity may be determined as described for Renilla luciferase activity, except 100 ml of LARII substrate from Promega will be used. Protein concentrations in cell lysates may be determined by Bradford Assay (Bio-Rad Laboratories, Hercules, CA). Renilla luciferase activities may be normalized for protein content and for transfection efficiency using firefly luciferase activity and will be expressed as relative light units (RLU) per microgram protein per minute of counting.

To measure activation of PR, an ELISA-based PR transactivation assay may be performed as per manufacturer's guidelines (Panomics). Briefly, the nuclear lysates of cells or tumors may be generated as described by the manufacturer. Binding of ligand (agonist or antagonist) such as P4 or RU486 induces a conformational change in the receptor, allowing the receptor to bind to specific DNA sites; progesterone response elements. Activated PR from nuclear extracts may be allowed to bind to the PR consensus binding site (PR probe) on a biotinylated oligonucleotide. These oligonucleotides may then be immobilized on a streptavidin-coated 96-well plate. The PR bound to the oligonucleotide may be detected by an antibody directed against PR. An additional horseradish peroxidase-conjugated secondary antibody may provide colorimetric readout quantified by reading absorbance at 450 nm.

Using PRE-Luciferase assay and further validated with ELISA-based PR transactivation, it is possible to determine the agonist and antagonist activities of the progesterone antagonists. If mixed activity is seen, pure antagonist compounds may be to tested to determine their efficacy in *in vivo* animal models. Further testing may be performed to determine the agonist and antagonist's activity on other reproductive tissues, especially for use in breast cancer treatment and prevention.

In this patent, certain U.S. patents, U.S. patent applications, and other materials (e.g., articles) have been incorporated by reference. The text of such U.S. patents, U.S. patent applications, and other materials is, however, only incorporated by reference to the extent that no conflict exists between such text and the other statements and drawings set forth herein. In the event of such conflict, then any such conflicting text in such incorporated by reference U.S. patents, U.S. patent applications, and other materials is specifically not incorporated by reference in this patent.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

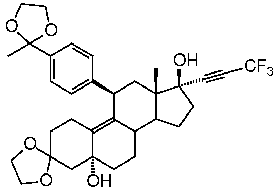

### 3,3-Ethylenedioxy-5α,17β-dihydroxy-17-(3,3,3-trifluoro-1-propynyl)-11β-{4'-[1',1'-(ethylenedioxy)-ethyl]phenyl}-estr-9-ene (6)

To a solution of diisopropylamine (21.6 mL, 154 mmol) in THF (40 mL) at -78° C under argon, n-butyllithium (55 mL, 2.5 N, 137.5 mmol) was added during 10 minutes and stirred for 30 minutes. Separately, a solution of 2-bromo-3,3,3-trifluoropropene (12 g, 68.5 mmol) in THF (80 mL) was made, cooled to -78° C and above prepared LDA was slowly added during 20 minutes. After stirring for 15 minutes, a solution of 3,3-ethylenedioxy-5α-hydroxy-11β-{4'-[1',1'-(ethylenedioxy)-ethyl]phenyl}-estra-9-ene-17-one (**5**) (6 g, 12.1 mmol) (Rao et al., Steroids, 1998, 63, 523) in THF (80 mL) was introduced during 15 minutes and stirred at -78° C for 1 h and slowly allowed to warm to room temperature during 15 hrs. Reaction mixture was quenched with aqueous ammonium chloride (50 mL) and extracted with ethyl acetate (3X100 mL). The combined organic layer was washed further with water and brine, dried over sodium sulfate and evaporated in vacuo to afford crude product. Purification was performed on a silica gel using 25% ethyl acetate in hexane to afford **6** (5.0 g, 70%).
¹H NMR (δ, 300 MHz) 0.45 (s, 3H), 1.63 (s, 3H), 1.1-2.5 (m, 19H), 3.7-4.1 (m, 8H), 4.34 (d, J=6.3 Hz, 1H), 4.44 (s, 1H), 7.17 (d, J=8.2 Hz, 2H), 7.34 (d, J=8.2 Hz).
¹³C NMR (75 MHz) 13.5, 23.4, 23.9, 24.1, 27.5, 35.1, 38.3, 38.7, 39.21, 39.25, 47.37, 47.49, 50.1, 59.54, 64.15, 64.53, 64.6, 64.76, 70.1, 74.1 (q, J=52 Hz) 80.0 (d, J=1.1 Hz), 90.5 (q, J=6.5 Hz), 108.7, 108.9, 114(q, J=255 Hz), 125.2, 127.0, 133.2, 135.1, 140.6, 146.2

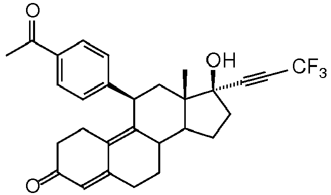

### 11β-(4'-Acetylphenyl)-17β-hydroxy-17-(3,3,3-trifluoro-1-propynyl)-estra-4,9-diene-3-one (1a)

To a solution of 3,3-ethylenedioxy-5α,17β-dihydroxy-17-(3,3,3-trifluoro-1-propynyl)-11β-{4'-[1',1'-(ethylenedioxy)-ethyl]phenyl}-estr-9-ene (**6**) (3.5 g, 6 mmol) in methanol (35 mL) at 0° C, 50% sulfuric acid (2.2 mL) was introduced and allowed to stir at room temperature for 2 hrs. The reaction mixture was carefully quenched with sodium bicarbonate solution (15 mL) and extracted with dichloromethane (3 X 20 mL). The combined organic layer was washed further with water and brine, dried over sodium sulfate and evaporated in vacuo to afford crude product. Purification was carried out on a silica gel using 25% ethyl acetate in hexane to afford **1a** (2.5 g, 87%).
¹H NMR (δ, 300 MHz) 0.52 (s, 3H), 1.3-2.9 (m, 17H), 2.58 (s, 3H), 4.0 (bs, 1H), 4.46 (d, J=7.1 Hz, 1H), 5.81 (s, 1H), 7.26 (d, J=8.3 Hz, 2H), 7.89 (d, J=8.4 Hz, 2H).
¹³C NMR (75 MHz) 13.6, 23.4, 25.8, 26.4, 27.3, 31.0, 36.5, 38.3, 39.071, 39.169, 40.6, 47.5, 50.1, 73.5 (q, J=52 Hz), 79.3 (d, J=1 Hz), 90.8 (q, J=6.3 Hz), 114.3 (q, J=256 Hz), 123.3, 126.8, 127.2, 128.8, 130.3, 134.9, 144.0, 150.4, 156.5, 197.9, 199.6.

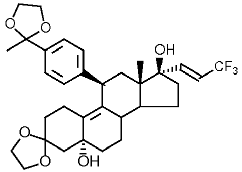

### 3,3-Ethylenedioxy-5α,17β-dihydroxy-17-(3,3,3-trifluoroprop-1(E)-enyl)-11β-{4'-[1'-1'-(ethylenedioxy)-ethyl]phenyl}-estr-9-ene (7)

To a slurry of 3,3-ethylenedioxy-5α,17β-dihydroxy-17-(3,3,3-trifluoro-1-propynyl)-11β-{4'-[1',1'-(ethylenedioxy)-ethyl]phenyl}-estr-9-ene (**6**) (1.2 g, 2 mmol) in ether (10 mL) and toluene (10 mL) at -78° C, sodium bis(2-metoxyethoxy) aluminum hydride solution ≥ 65% wt. in toluene (2.1 mL, 7.1 mmol) was introduced and allowed to stir for 3 hrs at -78° C. The reaction mixture was allowed to warm to room temperature during 1 hr. Reaction mixture was quenched with saturated ammonium chloride solution (20 mL) and extracted with ethyl acetate (3 X 20 mL). The combined organic layer was washed further with water and brine, dried over sodium sulfate and evaporated in vacuo to afford **7** (1.2 g crude product).
¹H NMR (δ, 300 MHz) 0.51 (s, 3H), 1.64 (s, 3H), 1.1-2.5 (m, 21H), 3.7-4.1 (m, 8H), 4.30 (d, J=6 Hz, 1H), 4.45 (s, 1H), 5.80-6.00 (m, 1H), 6.54 (d, J= 15.5 Hz, 1H), 7.19 (d, J=8.2 Hz, 2H), 7.34 (d, J=8.3 Hz, 2H).

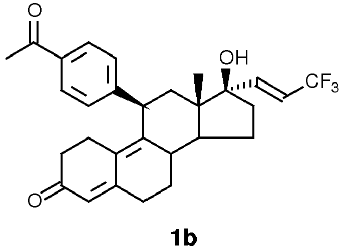

### 11β-(4'-Acetylphenyl)-17β-hydroxy-17-(3,3,3-trifluoroprop-1(E)-enyl)-estra-4,9-diene-3-one (1b)

Following the procedure outlined for the synthesis of compound **1a,** the hydrolysis of **7** (1.2 g) was carried out using 50% sulfuric acid to give after workup and purification **1b** (700 mg). ¹H NMR (δ, 300 MHz) 0.59 (s, 3H), 2.57 (s, 3H), 1.3-2.8 (m, 19H), 4.42 (d, J=7.0 Hz, 1H), 5.80 (s, 1H), 5.80-6.00 (m, 1H), 6.57 (d, J=15.5 Hz, 1H), 7.28 (d, J=8.0 Hz, 2H), 7.87 (d, J=8.2 Hz, 2H).

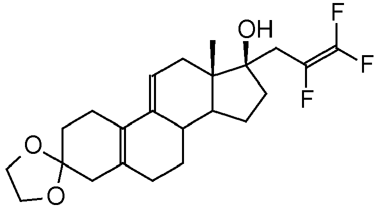

### 3,3-Ethylenedioxy-17β-hydroxy-17-(2,3,3-trifluoroprop-2-enyl)-5(10),9(11)-estradiene (9)

To a solution of 1,1,1,2-tetrafluoroethane (820 mg, 8 mmol) in ether (10 mL) at -78° C, n-BuLi (2.5 M, 2.4 mL, 6.1 mmol) was introduced during 10 minutes and allowed to stir for 1 h at -78° C. A solution of spiro-2'-(1'-oxacyc1opropane)-17(S)-[3,3-(ethylenedioxy)-5(10),9(11)-estradiene] (**8**) (1 g, 3.04 mmol) (Liu et al., J. Med. Chem., 1992, 35, 2113) in ether (7 mL) was introduced, followed by boron trifluoride etherate (0.38 mL, 3.04 mmol) dropwise. The reaction mixture was stirred at -78° C for 1 hr and allowed to warm to room temperature during 1 hr. Quenched with sodium bicarbonate solution (20 mL) and extracted with ethyl acetate (3 X 15 mL). The combined organic layer was washed further with water and brine, dried over sodium sulfate and evaporated in vacuo to afford crude product. Purification was carried out on a silica gel using 20% ethyl acetate in hexane to afford 9 (430 mg, 35%).
¹H NMR (δ, 300 MHz) 0.90 (s, 3H), 1.0-2.7 (m, 20H), 3.99 (s, 4H), 5.50-5.60 (bs, 1H).
¹³C NMR (75 MHz) 14.4, 23.6, 24.6, 27.6, 31.2, 31.3, 32.8, 33.7 (dd, J=2.8, 18.6 Hz), 34.7, 39.4, 41.3, 45.3, 46.2, 46.8, 64.36, 64.49, 82 (m), 108.2, 117.7, 126.1, 125-130 (m), 130.2, 136.5, 154.8 (ddd, J=285, 271, 47.2 Hz).

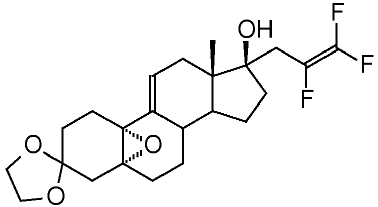

### 3,3-Ethylenedioxy-5α,10α-epoxy-17β-hydroxy-17-(2,3,3-trifluoroprop-2-enyl)-estr-9(11)-ene (10)

Hydrogen peroxide (0.18 mL, 30%, 1.6 mmol) was added to an ice-cold solution of hexafluoroacetone trihydrate (350 mg, 1.6 mmol) in dichloromethane (3 mL). Solid Na₂HPO₄ (180 mg, 1.3 mmol) was introduced and the reaction mixture was stirred for 1 hr at 0° C. An ice-cold solution of 3,3-ethylenedioxy-17β-hydroxy-17-(2,3,3-trifluoroprop-2-enyl)-5(10),9(11)-estradiene (**9**) (410 mg, 1 mmol) in dichloromethane (3 mL) was added and the mixture was stirred at 0° C for 3 hrs then at 5° C for 15 hrs. The reaction mixture was diluted with dichloromethane (15 mL) and washed with 10% sodium sulfite solution (15 mL), water, dried over sodium sulfate and concentrated under vacuum to obtain the mixture of crude epoxides. Separation of isomeric epoxide was carried out on a silica gel column using 20% ethyl acetate in hexane to afford **10** (240 mg, 56%) of pure α-isomer.
¹H NMR (δ, 300 MHz) 0.9 (s, 3H), 1.0-2.8 (m, 20H), 3.8-4.0 (m, 4H), 5.90-6.10 (m, 1H).

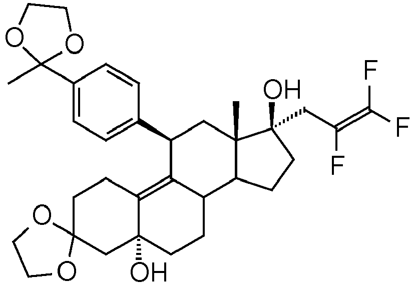

### 3,3-Ethylenedioxy-5α,17β-dihydroxy-17-(2,3,3-trifluoroprop-2-enyl)-11β-{4'-[1'-1'-(ethylenedioxy)-ethyl]phenyl}-estr-9-ene (11)

A slurry of magnesium (220 mg, 9 mmol) in THF (10 mL) containing a crystal of iodine was taken and heated to reflux for 10 minutes to become colorless. A solution of 2-(4-bromophenyl)-2-methyl-1,3-dioxolane (2.1 g, 8.5 mmol) in THF (5 mL) was introduced during 5 minutes and allowed to reflux for 1 hr. Reaction mixture was cooled under ice and solid CuCl (150 mg, 1.5 mmol) was added to it and continued to stir at 0° C for 30 minutes. Finally a solution of 3,3-ethylenedioxy-5α,10α-epoxy-17β-hydroxy-17-(2,3,3-trifluoroprop-2-enyl)-estr-9(11)-ene (**10**) (730 mg, 1.7 mmol) in THF (5 mL) was added into the cuprate solution and allowed to stir for 2 hrs at 0° C. Quenched with aqueous ammonium chloride solution (30 mL) and extracted with ethyl acetate (3X 25 mL). The combined organic layer was washed further with water and brine, dried over sodium sulfate and evaporated in vacuo to afford crude product. Purification was carried out on a silica gel using 25% ethyl acetate in hexane to afford **11** (810 mg, 80%).
¹H NMR (δ, 300 MHz) 0.48 (s, 3H), 0.8-2.7 (m, 24H), 3.6-4.6 (m, 10H), 6.79 (d, J=8.8 Hz, 1H), 7.18 (d, J=8.2 Hz, 2H), 7.30-7.40 (m, 3H).

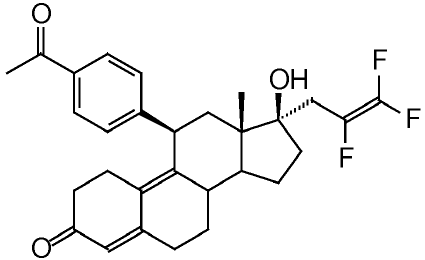

### 11β-(4'-Acetylphenyl)-17β-hydroxy-17-(2,3,3-trifluoroprop-2-enyl)-estra-4,9-diene-3-one (1c)

Following the procedure outlined for the synthesis of compound **1a,** the hydrolysis of **11** (1.5 g) was carried out using 50% sulfuric acid to give after workup and purification **1c** (1.1 g). ¹H NMR (δ, 300 MHz) 0.56 (s, 3H), 1.0-2.8 (m, 22H), 4.48 (d, J=6.7 Hz, 1H), 5.80 (s, 1H), 7.29 (d, J=8.4 Hz, 2H), 7.88 (d, J=8.4 Hz, 2H).
¹³C NMR (75 MHz) 15.2, 22.7, 23.5, 25.8, 26.4, 27.4, 30.9, 33.5 (d, J=18.7 Hz), 34.3, 36.6, 37.1, 39.3, 40.5, 46.7, 50.0, 82.8 (d, J=2.7 Hz), 123.4, 125-130 (m), 127.0, 128.6, 130.2, 134.9, 143.9, 150.0, 154.7 (ddd, J=47, 272, 286 Hz), 155.9, 197.4, 198.9.

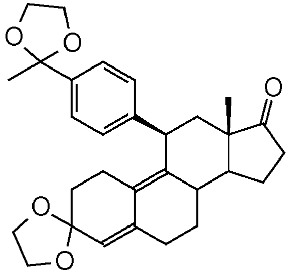

### 3,3-Ethylenedioxy-11β-{4'-[1',1'-(ethylenedioxy)-ethyl]phenyl}-estra-4,9-diene-17-one (12)

To a solution of 3,3-ethylenedioxy-5α-hydroxy-11β-{4'-[1',1'-(ethylenedioxy)-ethyl]phenyl}-estra-9-ene-17-one (**5**) (3 g, 6 mmol) in pyridine (30 mL), DMAP (150 mg) and acetic anhydride (3 mL) were added and heated at 70° C for 30 hrs. The reaction mixture was concentrated under vacuum and directly purified on a silica gel using 10% ethyl acetate in hexane containing 1% TEA to afford **12** (2.3 g, 80%).
¹H NMR (δ, 300 MHz) 0.48 (s, 3H), 1.2-2.8 (m, 17H), 1.63 (s, 3H), 3.7-4.1 (m, 8H), 4.31 (d, J=7.1 Hz, 1H), 5.4 (s, 1H), 7.18 (d, J=8.2 Hz, 2H), 7.34 (d, J=8.3 Hz, 2H).
¹³C NMR (75 MHz) 14.5, 22.0, 24.3, 27.4, 27.5, 30.5, 33.2, 35.5, 37.5, 37.7, 39.6, 47.8, 51.0, 64.5, 64.6, 64.7, 106.2, 108.9, 121.9, 125.4, 127.0, 130.4, 137.5, 139.3, 140.7, 144.6, 219.6.

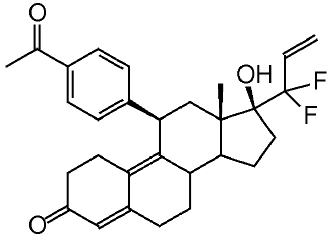

### 11β-(4'-Acetylphenyl)-17β-hydroxy-17-(1,1-difluoroprop-2-enyl)-estra-4,9-diene-3-one (1d)

To a solution of 3,3-ethylenedioxy-11β-{4'-[1',1'-(ethylenedioxy)-ethyl]phenyl}-estra-4,9-diene-17-one (**12**) (400 mg, 0.84 mmol) and 3-bromo-3,3-difluoropropene (530 mg, 3.4 mmol) in a (4:1:1) THF-ether-pentane mixture (6 mL) at -100° C, n-BuLi (1.3 mL, 2.5 M, 3.2 mmol) was added dropwise. The reaction mixture was allowed to stir for 90 minutes at -95° C and allowed to warm to room temperature over 3 hrs. Quenched with ammonium chloride solution (20 mL) and extracted with ethyl acetate (3 X 15 mL). The combined organic layer was concentrated to dryness, dissolved in methanol (5 mL) and treated with 50% sulfuric acid (0.25 mL) at 0° C. Reaction was allowed to stir at room temperature for 2 hrs and carefully quenched with sodium bicarbonate solution (15 mL). Organic materials were extracted with dichloromethane (3 X 10 mL) and the combined dichloromethane layers were dried over sodium sulfate, concentrated under vacuum. Purification was effected on a silica gel column using 25 % ethyl acetate in hexane to afford **1d** (160 mg, 40%).
¹H NMR (δ, 300 MHz) 0.52 (s, 3H), 1.2-2.8 (m, 17H), 2.52 (s, 3H), 4.42 (bs, 1H), 5.50 (d, J=11.1 Hz, 1H), 5.68 (d, J=17.3 Hz, 1H), 5.74 (s, 1H), 6.0-6.3 (m, 1H), 7.26 (d, J=8.1 Hz, 2H), 7.83 (d, J=8.3 Hz, 2H).
¹³C NMR (75 MHz) 16.9, 24.5, 25.8, 26.5, 27.7, 31.1, 33.6, 36.7, 38.8, 39.5, 41.0, 48.2, 51.0, 85.1 (t, J=26 Hz), 120.7 (t, J=9.5 Hz), 123.0 (t, J=247 Hz), 123.2, 127.1, 128.7, 129.9, 131.2 (t, J=25.2 Hz), 134.9, 144.3, 150.7, 156.3, 197.7, 199.3.

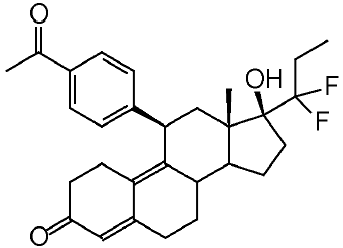

### 11β-(4'-Acetyl-phenyl)-17β-hydroxy-17-(1,1-difluoropropyl)-estra-4,9-diene-3-one (1e) (comparative example)

To a solution of 11β-(4'-acetyl-phenyl)-17β-hydroxy-17-(1,1-difluoroprop-2-enyl)-estra-4,9-diene-3-one (**1d**) (160 mg, 0.34 mmol) in ethanol (5 mL) containing 10% Pd/C (20 mg) was stirred under balloon pressure of hydrogen for 2 hrs. Catalyst was filtered through cotton plug, solvents were removed under vacuum and purified on a silica gel column to yield **1e** (120 mg, 75%).
¹H NMR (δ, 300 MHz) 0.53 (s, 3H), 1.03 (t, J=7.4 Hz, 3H), 1.1-2.8 (m, 19H), 2.55 (s, 3H), 4.44 (bs, 1H), 5.76 (s, 1H), 7.28 (d, J=8.4 Hz), 7.86 (d, J=8.4 Hz).
¹³C NMR (75 MHz) 1.0, 5.5 (t, J=6.1 Hz), 17.1, 24.7, 25.9, 26.1, 26.6, 27.7, 31.1, 34.1, 36.8, 39.1, 39.4, 41.2, 48.6, 51.4, 85.7 (t, J=26 Hz), 123.3, 127.2, 127.8 (t, J=249 Hz), 128.8, 130.0, 135.0, 144.3, 150.9, 156.3, 197.7, 199.3.

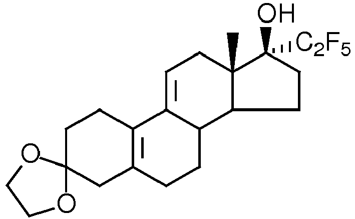

### 3,3-Ethylenedioxy-17β-hydroxy-17-(1,1,2,2,2-pentafluoroethyl)-estra-5(10),9(11)-diene (14)

To a solution of 3,3-ethylenedioxy-estra-5(10),9(11)-diene-17-one (**13**) (2.5 g, 8 mmol) in toluene (32 mL) at -78° C, pentafluoroiodoethane (4 g, 16 mmol) was condensed and allowed to stir for 10 min. Methyllithium lithium bromide solution in ether (1.5M, 9.8 mL) was introduced dropwise during 5 min and continued to stir at -78° C for 1 hr. Reaction mixture was warmed to 0° C and stirred for 1 hr under ice before quenching with water. Extracted with ethyl acetate (2X25 mL) and the combined organic layer was washed once with brine, dried over sodium sulfate, concentrated under reduced pressure to obtain **14** (3.0 g, 85%), which was used for epoxidation without further purification.
¹H NMR (δ, 300 MHz) 0.93 (s, 3H), 1.2-2.8 (m, 18H), 3.98(s, 4H), 5.6 (bs, 1H).

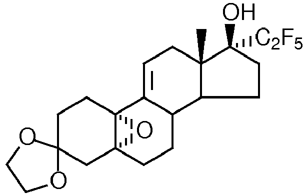

### 3,3-Ethylenedioxy-5α,10α-epoxy-17β-hydroxy-17-(1,1,2,2,2-pentafluoroethyl)-estra-9(11)-ene (15)

Following the procedure outlined for the synthesis of compound **10,** the epoxidation of **14** (3.0 g) was carried out using hydrogen peroxide and hexafluoroacetone in dichloromethane and gave after workup and purification **15** (1.8 g).
¹H NMR (δ, 300 MHz) 0.92 (s, 3H), 1.1-2.8 (m, 18H), 3.70-4.00 (m, 4H), 6.0 (bs, 1H).
¹³C NMR (75 MHz) 15.8, 22.8, 25.1, 25.2, 28.1, 31.8, 34.4, 35.4 (dd, J = 3.6, 8.1 Hz), 38.4, 40.3, 48.8, 49.0, 49.1, 60.1, 61.7, 64.1, 64.4, 84.2 (dd, J = 25.1, 21.5 Hz), 107.1, 117 (m), 122 (m), 126.86, 126.89, 135.1

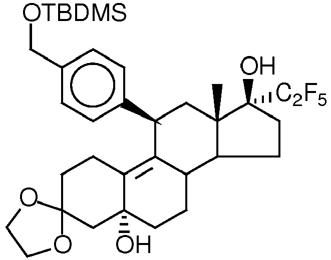

### 3,3-Ethylenedioxy-5α,17β-dihydroxy-17-(1,1,2,2,2-pentafluoroethyl)-11β-[4'-(tert-butyldimethylsilyloxymethyl)phenyl] -estr-9-ene (16)

Following the procedure outlined for the synthesis of compound **11,** the Grignard reaction of **15** (230 mg) was carried out using 4-(t-butyldimethylsilyloxymethyl) bromobenzene and magnesium and gave after workup and purification the required product **16** (340 mg).
¹H NMR (δ, 300 MHz) 0.08 (s, 6H), 0.53 (s, 3H), 0.93 (s, 9H), 1.2-2.5 (m, 18H), 3.8-4.1 (m, 4H), 4.4 (bd, 1H), 4.70 (s, 2H), 7.0-7.2 (m, 4H).

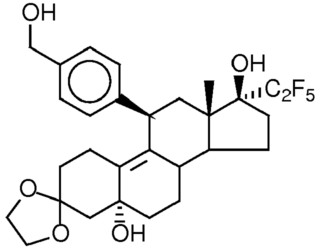

### 3,3-Ethylenedioxy-5α,17β-dihydroxy-17-(1,1,2,2,2-pentafluoroethyl)-11β-[4'-(hydroxymethyl) phenyl]-estr-9-ene (17)

To a solution of 3,3-ethylenedioxy-5α,17β-dihydroxy-17-(1,1,2,2,2-pentafluoroethyl)-11β-[4'-(*tert*-butyldimethylsilyloxymethyl)phenyl]-estr-9-ene (**16**) (340 mg) in THF (3 mL) under argon, TBAF (1.0 M, 1.3 mL) was introduced and stirred at rt for 2 hrs. Solvents were removed under reduced pressure and directly purified on a silica gel column using 60% ethyl acetate in hexane to afford **17** (210 mg, 75%).

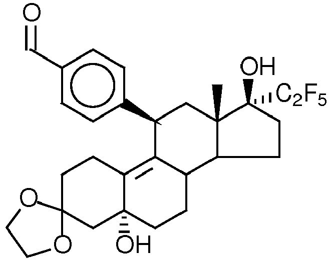

### 3,3-Ethylenedioxy-5α,17β-dihydroxy-17-(1,1,2,2,2-pentafluoroethyl)-11β-(4'-formylphenyl)-estr-9-ene (18)

To a slurry of 3,3-ethylenedioxy-5α,17β-dihydroxy-17-(1,1,2,2,2-pentafluoroethyl)-11β-[4'-(hydroxymethyl) phenyl]-estr-9-ene (17) (210 mg), NMO (66 mg) and powdered 4A molecular sieve (190 mg) in dichloromethane (8 mL), TPAP (7 mg) was introduced at once and stirred at rt for 2 hrs. Pure product was obtained by directly passing through a silica gel column using 30% ethyl acetate in hexane to afford 200 mg of **18.**
¹H NMR (δ, 300 MHz) 0.51 (s, 3H), 1.0-2.8 (m, 18H), 3.8-4.1 (m, 4H), 4.2-4.4 (m, 2H), 7.41 (d, J = 8.1 Hz, 2H), 7.78 (d, J = 8.3 Hz, 2H), 9.96 (s, 1H).

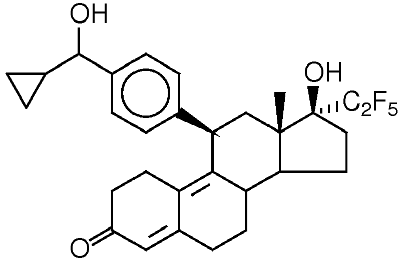

### 11β-(4'-[1-cyclopropyl-hydroxymethyl]phenyl)-17β-hydroxy-17-(1,1,2,2,2-pentafluoroethyl)-estra-4,9-diene-3-one (20)

To a solution of 3,3-ethylenedioxy-5α,17β-dihydroxy-17-(1,1,2,2,2-pentafluoroethyl)-11β-(4'-formylphenyl)-estr-9-ene (**18**) (650 mg) in THF (16 mL) at -5° C, cyclopropyl magnesium bromide (0.5 M, 9.3 mL) was added and stirred at rt for 1hr. Quenched with water and extracted with ethyl acetate (3X20 mL). The combined organic layer was washed once with brine, dried over sodium sulfate, concentrated under reduced pressure to obtain the crude product. Hydrolysis of the crude alcohol **19** was carried out using 50% sulfuric acid, according to procedure detailed for **1a.** Purification was effected on a silica gel column using 30% ethyl acetate in hexane to obtain 500 mg of **20** (80%)
¹H NMR (δ, 300 MHz) 0.2-0.3 (m, 1H), 0.4-0.5 (m, 2H), 0.60 (s, 3H), 0.5-0.7 (m, 1H), 1.0-1.2 (m, 1H), 1.3-2.8 (m, 16H), 3.24 (s, 3H), 3.53 (d, J = 8 Hz, 1H), 4.46 (d, J = 6.7 Hz, 1H), 5.78 (s, 1H), 7.14 (d, J = 8.0 Hz, 2H), 7.23 (d, J = 8.2 Hz, 2H).
¹³C NMR (75 MHz) 1.6, 4.12, 4.15, 16.4, 16.98, 17.04, 24.8, 25.68, 27.5, 30.9, 33.0, 36.5, 38.49, 38.6, 39.11, 39.14, 40.45, 50.36, 51.64, 51.70, 56.42, 84.1 (dd, J = 24.8, 21.2 Hz), 86.99, 87.01, 117 (m), 121 (m), 122.9, 126.5, 126.9, 129.6, 139.1, 143.2, 144.6, 156.4, 199.5.

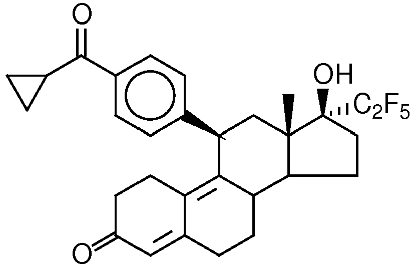

### 11β-(4'-[Cyclopropylcarbonyl]phenyl)-17β-hydroxy-17-(1,1,2,2,2-pentafluoroethyl)-estra-4,9-diene-3-one (comparative example)

To a slurry of 11β-(4'-[1-cyclopropyl-hydroxymethyl]phenyl)-17β-hydroxy-17-(1,1,2,2,2-pentafluoroethyl)-estra-4,9-diene-3-one (**20**) (500 mg) and molecular sieve 3A (500 mg) in dichloromethane (10 mL), PCC (800 mg) was added and stirred over night. Purification was directly effected on a silica gel column using 20% ethyl acetate in hexane to afford 350 mg (70%) of **2a.**
¹H NMR (δ, 300 MHz) 0.58 (s, 3H), 1.0-2.9 (m, 21H), 4.48 (d, J = 6.9 Hz, 1H), 5.78 (s, 1H), 7.30 (d, J = 8.2 Hz, 2H), 7.92 (d, J = 8.2 Hz, 2H).

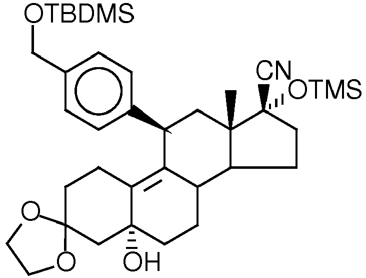

### 3,3-Ethylenedioxy-17β-cyano-5α-hydroxy-17α-trimethylsilyloxy-11β-[4'-(tert-butyldimethylsilyloxymethyl)phenyl]-estr-9-ene (22)

Following the procedure outlined for the synthesis of compound **11,** the Grignard reaction of **21** (15g) was carried out using 4-(t-butyldimethylsilyloxymethyl) bromobenzene and magnesium and gave after workup and purification **22** (15.4 g).

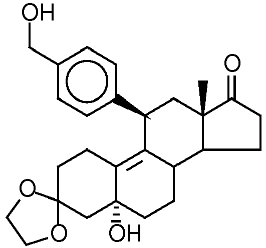

### 3,3-Ethylenedioxy-5α-hydroxy-11β-[4'-(hydroxymethyl)phenyl]-estr-9-ene-17-one (23)

To a solution of 3,3-ethylenedioxy-17β-cyano-5α-hydroxy-17α-trimethylsilyloxy-11β-[4'-(*tert-*butyldimethylsilyloxymethyl)phenyl]-estr-9-ene (**22**) (15.4 g) in THF (150 mL), a solution of TBAF (1M, 59 mL) in THF was introduced dropwise and allowed to stir for 1h. THF was removed under reduced pressure and the resulting oil was suspended in a mixture of water (90 mL), methanol (10 mL) and CH₂Cl₂ (10 mL) and cooled to 0° C. Sodium hydroxide solution (2M, 80 mL) was introduced and allowed to stir at rt for 2 hrs. Finally, water (50 mL) was added to the reaction mixture and extracted with dichloromethane (3X50 mL). The combined organic layer was washed once with water (30 mL), dried over sodium sulfate and concentrated under reduce pressure to obtain the crude product. Purification was carried out on a silica gel column using 5% acetone in dichloromethane to afford 10 g of (**23**).
¹H NMR (δ, 300 MHz) 0.49 (s, 3H), 1.0-2.8 (m, 18H), 3.8-4.1 (m, 4H), 4.20-4.35 (m, 1H), 4.40 (s, 1H), 4.60-4.70 (m, 2H), 7.2-7.4 (m, 4H).

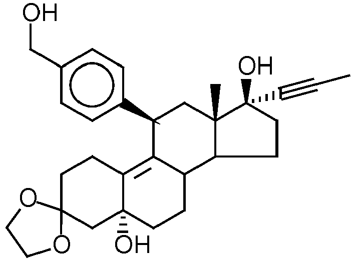

### 3,3-Ethylenedioxy-5α,17β-dihydroxy-17-(1-propynyl)-11β-[4'-(hydroxymethyl)phenyl]-estr-9-ene (24)

To a solution of 3,3-ethylenedioxy-5α-hydroxy-11β-[4'-(hydroxymethyl)phenyl]-estr-9-ene-17-one (**23**) (2 g) in THF (40 mL), propynyl magnesium bromide (0.5 M, 44 mL) was added dropwise and allowed to stir at 50° C for 2 hrs. Reaction was quenched with NaHCO₃ (20 mL) and extracted with ethyl acetate (3X20 mL). The combined organic layer was washed once with water (20 mL), dried over sodium sulfate and concentrated under reduced pressure to obtain the crude product. The crude material was triturated with dichloromethane to obtain 1.45 g of **24.**
¹H NMR (δ, 300 MHz) 0.45 (s, 3H), 1.0-2.6 (m, 18H), 1.89 (s, 3H), 3.8-4.1 (m, 4H), 4.2-4.5 (m, 2H), 4.60-4.70 (m, 2H), 7.10-7.35 (m, 4H).

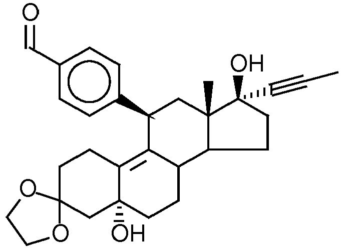

### 3,3-Ethylenedioxy-5α,17β-dihydroxy-17-(1-propynyl)-11β-(4'-formylphenyl)-estr-9-ene (25)

Following the procedure outlined for the synthesis of compound **18,** the oxidation of **24** (1.42 g) was carried out using TPAP and NMO and gave after workup and purification the required product **25** in quantitative yields.
¹H NMR (δ, 300 MHz) 0.42 (s, 3H), 1.1-2.6 (m, 18H), 1.89 (s, 3H), 3.8-4.1 (m, 4H), 4.3-4.4 (m, 1H), 4.45 (s, 1H), 7.41 (d, J = 8.1 Hz, 2H), 7.78 (d, J = 8.3 Hz, 2H), 9.96 (s, 1H).

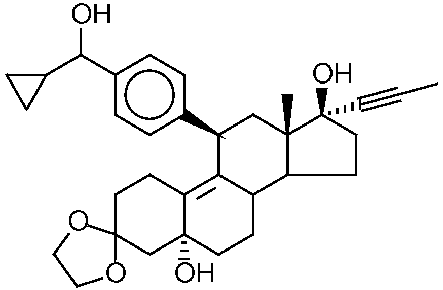

### 3,3-Ethylenedioxy-5α,17β-dihydroxy-17-(1-propynyl)-11β-(4'-[1-cyclopropyl-hydroxymethyl] phenyl)-estr-9-ene (26)

To a solution of 3,3-ethylenedioxy-5α,17β-dihydroxy-17-(1-propynyl)-11β-(4'-formylphenyl)-estr-9-ene (**25**) (1.4 g) in THF (35 mL) at -5° C, cyclopropyl magnesium bromide (0.5 M, 23.5 mL) was added and stirred at rt for 1hr. Quenched with water and extracted with ethyl acetate (3X20 mL). The combined organic layer was washed once with water (20 mL), dried over sodium sulfate and concentrated under reduced pressure to obtain the crude product. Purification was performed on a silica gel column to obtain 750 mg of **26.**
¹H NMR (δ, 300 MHz) 0.44 (s, 3H), 0.2-0.8 (m, 5H), 1.0-2.6 (m, 18H), 1.90 (s, 1H), 3.8-4.1 (m, 4H), 4.2-4.4 (m, 1H), 4.45 (s, 1H), 7.19 (d, J = 7.8 Hz, 2H), 7.30 (d, J = 8 Hz, 2H).

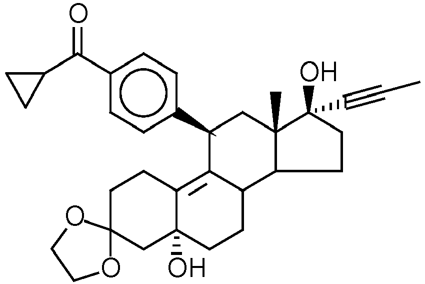

### 3,3-Ethylenedioxy-5α,17β-dihydroxy-17-(1-propynyl)-11β-(4'-[cyclopropylcarbonyl]phenyl)-estr-9-ene (27)

Following the procedure outlined for the synthesis of compound **18,** the oxidation of **26** was carried out using TPAP and NMO and gave after workup and purification the required product **27** in quantitative yields.
¹H NMR (δ, 300 MHz) 0.44 (s, 3H), 1.0-2.7 (m, 23H), 1.9 (s, 3H), 3.6-4.1 (m, 4H), 4.30-4.40 (m, 1H), 4.45 (s, 1H), 7.34 (d, J = 8.4 Hz, 2H), 7.92 (d, J = 8.3 Hz, 2H).

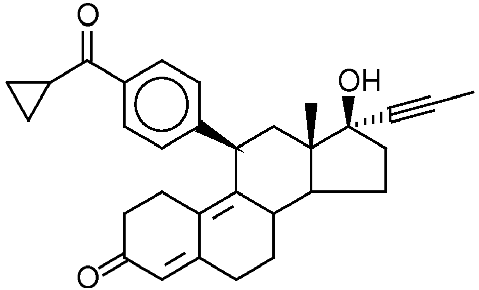

### 11β-(4'-[Cyclopropylcarbonyl]phenyl)-17β-hydroxy-17-(1-propynyl)-estra-4,9-diene-3-one (2b) (comparative example)

Following the procedure outlined for the synthesis of compound **1a,** the hydrolysis of **27** (690 mg) was carried out using 50% sulfuric acid to give after workup and purification **2b** (460 mg). ¹H NMR (δ, 300 MHz) 0.5 (s, 3H), 1.00-1.15 (m, 2H), 1.20-3.00 (m, 19H), 1.91 (s, 3H), 4.4-4.5 (m, 1H), 5.8 (s, 1H), 7.31 (d, J = 7.7 Hz, 2H), 7.95 (d, J = 7.8 Hz, 2H).

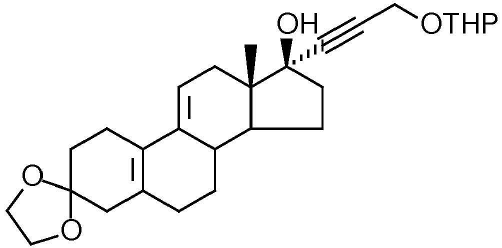

### 3,3-Ethylenedioxy-17β-hydroxy-17-(3-tetrahydropyranyloxy-1-propynyl)-estra-5(10),9(11)-diene (28)

To a solution of tetrahydro-2-(2-propynyloxy)-2H-pyran (13.4 g) in THF (50 mL) at 0° C, n-BuLi (2.5 M solution in hexane, 38 mL) was added dropwise and allowed to stir for 30 min at 0° C. A solution of 3,3-ethylenedioxy-estra-5(10),9(11)-diene-17-one (**13**) (10 g) in THF (50 mL) was added dropwise to the reaction mixture and allowed to stir for 2 hrs at 0° C. Quenched with saturated ammonium chloride solution (100 mL) and extracted with ethyl acetate (3 X 25 mL). The collective organic layer was washed once with water, dried over sodium sulfate and concentrated under reduced pressure. Purification on silica gel column yielded **28** (13 g).
¹H NMR (δ, 300 MHz) 0.84 (s, 3H), 1.10-2.8 (m, 24H), 3.4-3.6 (m, 1H), 3.7-3.9 (m, 1H), 4.00 (s, 4H), 4.20-4.40 (m, 2H), 4.80 (s, 1H), 5.61 (s, 1H).

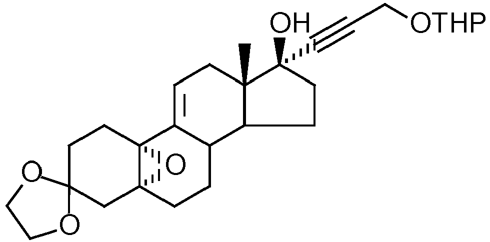

### 3,3-Ethylenedioxy-5α,10α-epoxy-17β-hydroxy-17-(3-tetrahydropyranyloxy-1-propynyl)-estra-9(11)-ene (29)

Following the procedure outlined for the synthesis of compound **10,** the epoxidation of **28** (10 g) was carried out using hydrogen peroxide and hexafluoroacetone in dichloromethane and gave after workup and purification **29** (5.2 g).
¹H NMR (δ, 300 MHz) 0.84 (s, 3H), 1.0-2.8 (m, 24H), 3.4-3.6 (m, 1H), 3.7-4.1 (m, 5H), 4.20-4.40 (m, 2H), 4.79 (s, 1H), 6.07 (s, 1H).

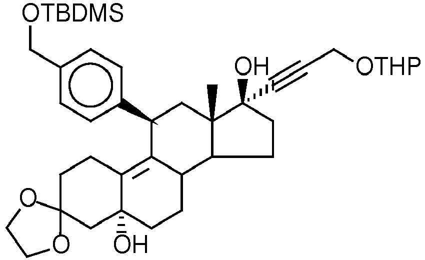

### 3,3-Ethylenedioxy-5α,17β-dihydroxy-17-(3-tetrahydropyranyloxy-1-propynyl)-11β-[4'-(tert-butyldimethylsilyloxymethyl)phenyl] -estr-9-ene (30)

Following the procedure outlined for the synthesis of compound **11,** the Grignard reaction of **29** (5.2 g) was carried out using 4-(t-butyldimethylsilyloxymethyl) bromobenzene and magnesium and gave after workup and purification **30** (5.8 g).
¹H NMR (δ, 300 MHz) 0.09 (s, 6H), 0.46 (s, 3H), 0.94 (s, 9H), 1.10-2.60 (m, 24H), 3.5-3.7 (m, 1H), 3.75-4.10 (m, 5H), 4.25-4.50 (m, 3H), 4.70 (s, 2H), 4.80 (s, 1H), 7.10-7.30 (m, 4H).

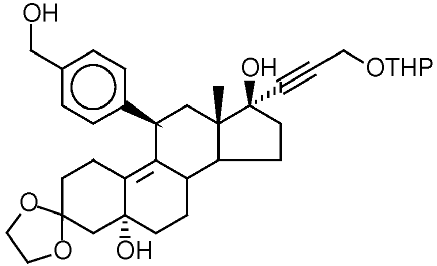

### 3,3-Ethylenedioxy-5α,17β-dihydroxy-17-(3-tetrahydropyranyloxy-1-propynyl)-11β-[4'-(hydroxymethyl)phenyl]-estr-9-ene (31)

Following the procedure outlined for the synthesis of compound **17,** the deprotection of **30** (5.8 g) was carried out using TBAF, gave after workup and purification **31** (4.7 g).
¹H NMR (δ, 300 MHz) 0.47 (s, 3H), 1.0-2.5 (m, 24H), 3.40-3.60 (m, 1H), 3.75-4.20 (m, 5H), 4.20-4.50 (m, 3H), 4.66 (s, 2H), 4.83 (s, 1H), 7.10-7.30 (m, 4H).

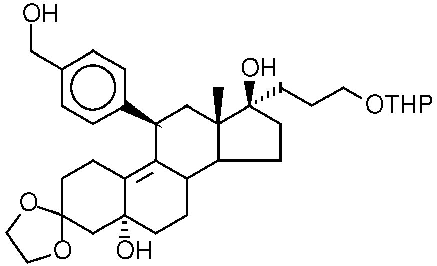

### 3,3-Ethylenedioxy-5α,17β-dihydroxy-17-(3-tetrahydropyranyloxy-1-propyl)-11β-[4'-(hydroxymethyl)phenyl]-estr-9-ene (32)

A solution of **31** (2.0 g) in THF (20 mL) was hydrogenated in a Paar apparatus using 5% Pd/C (190 mg) under 15 psi pressure of hydrogen for overnight. Catalyst was filtered off and washed with ethyl acetate (25 mL). The combined organic layer was concentrated under reduced pressure to obtain the crude product **32** (1.9 g).
¹H NMR (δ, 300 MHz) 0.48 (s, 3H), 1.10-2.60 (m, 28H), 3.30-3.60 (m, 2H), 3.70-4.10 (m, 6H), 4.20-4.40 (m, 2H), 4.50-4.70 (m, 3H), 7.10-7.30 (m, 4H).

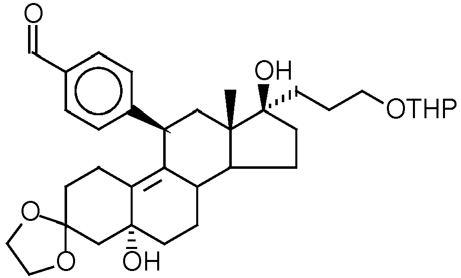

### 3,3-Ethylenedioxy-5α,17β-dihydroxy-17-(3-tetrahydropyranyloxy-1-propyl)-11β-(4'-formylphenyl)-estr-9-ene (33)

Following the procedure outlined for the synthesis of compound **18,** the oxidation of **32** (1.9 g) was carried out using TPAP and NMO and gave after workup and purification **33** (1.6 g).
¹H NMR (δ, 300 MHz) 0.46 (s, 3H), 1.10-2.6 (m, 28H), 3.30-3.60 (m, 2H), 3.60-4.10 (m, 5H), 4.38 (s, 2H), 4.60 (s, 1H), 7.42 (d, J = 8.2 Hz, 2H), 7.77(d, J = 8.2 Hz, 2H).

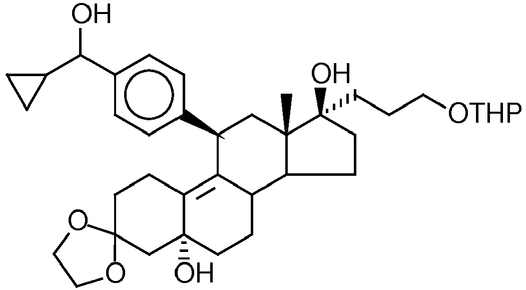

### 3,3-Ethylenedioxy-5α,17β-dihydroxy-17-(3-tetrahydropyranyloxy-1-propyl)-11β-(4'-[1-cyclopropyl-hydroxymethyl] phenyl)-estr-9-ene (34)

Following the procedure outlined for the synthesis of compound **26,** the cyclopropyl addition of **33** (1.55 g) was carried out using 4 equivalents of cyclopropyl magnesium bromide and gave after workup and purification **34** (XX g).
¹H NMR (δ, 300 MHz) 0.2-0.8 (m, 7H), 0.8-2.6 (m, 29H), 3.30-3.60 (m, 2H), 3.60-4.20 (m, 6H), 4.20-4.40 (m, 2H), 4.61 (s, 1H), 7.10-7.40 (m, 4H).

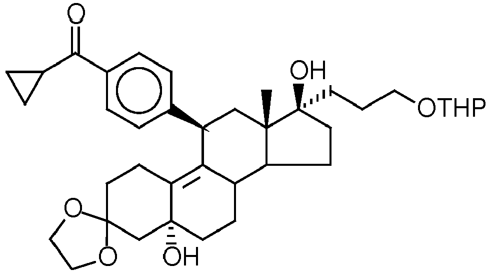

### 3,3-Ethylenedioxy-5α,17β-dihydroxy-17-(3-tetrahydropyranyloxy-1-propyl)-11β-(4'-[cyclopropylcarbonyl] phenyl)-estr-9-ene (35)

Following the procedure outlined for the synthesis of compound **18,** the oxidation of **34** (XX g) was carried out using TPAP and NMO and gave after workup and purification **35** (XX g).
¹H NMR (δ, 300 MHz) 0.49 (s, 3H), 0.9-2.80 (m, 32H), 3.30-3.60 (m, 2H), 3.60-4.20 (m, 6H), 4.40 (bs, 2H), 4.62 (s, 1H), 7.30 (d, J = 8.2 Hz, 2H), 7.93 (d, J = 8.3 Hz, 2H).

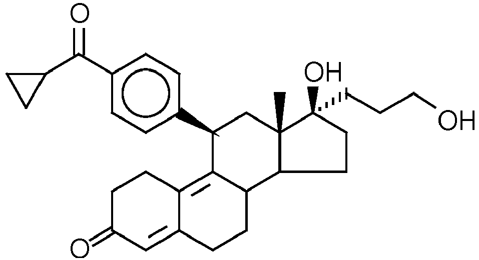

### 11β-(4'-[Cyclopropylcarbonyl]phenyl)-17β-hydroxy-17-(3-hydroxypropyl)-estra-4,9-diene-3-one (2c) (comparative example)

Following the procedure outlined for the synthesis of compound **1a,** the hydrolysis of **35** (XX g) was carried out using 50% sulfuric acid to give after workup and purification **2c** (XX g).
¹H NMR (δ, 300 MHz) 0.53 (s, 3H), 1.0-1.1 (m, 2H), 1.15-1.25 (m, 2H), 1.25-2.8 (m, 20H), 3.50-3.80 (m, 2H), 4.30-4.55 (m, 1H), 5.79 (s, 1H), 7.28 (d, J = 8.3 Hz, 2H), 7.93 (d, J = 8.4 Hz, 2H).

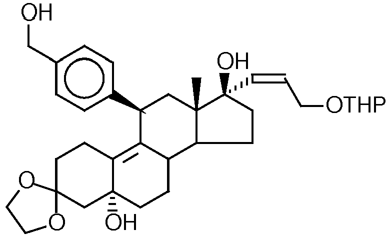

### 3,3-Ethylenedioxy-5α,17β-dihydroxy-17-(3-tetrahydropyranyloxy-prop-1(Z)-enyl)-11β-[4'-(hydroxymethyl)phenyl]-estr-9-ene (36)

A solution of 3,3-ethylenedioxy-5α,17β-dihydroxy-17-(3-tetrahydropyranyloxy-1-propynyl)-11β-[4'-(hydroxymethyl)phenyl]-estr-9-ene (**31**) (2.2 g) in ethanol (40 mL) containing 5% Pd/BaSO₄ (0.2 g) and pyridine (2 mL) was hydrogenated using balloon pressure of hydrogen and continuously monitored by TLC. Upon completion, catalyst was filtered and washed with ethyl acetate (30 mL). The combined filtrate was concentrated under reduced pressure to obtain the crude **36** (2.1 g).
¹H NMR (δ, 300 MHz) 0.50 (s, 3H), 1.0-2.8 (m, 24H), 3.30-3.60 (m, 2H), 3.70-4.15 (m, 5H), 4.20-4.80 (m, 6H), 5.50-5.80 (m, 2H), 7.10-7.30 (m, 4H).

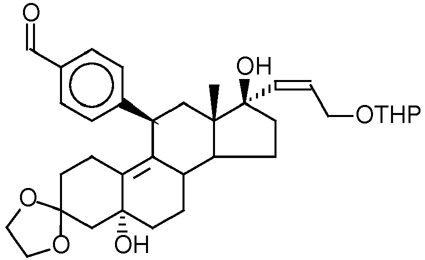

### 3,3-Ethylenedioxy-5α,17β-dihydroxy-17-(3-tetrahydropyranyloxy-prop-1(Z)-enyl)-11β-(4'-formylphenyl)-estr-9-ene (37)

Following the procedure outlined for the synthesis of compound **18,** the oxidation of **36** (2.5 g) was carried out using TPAP and NMO and gave after workup and purification **37** (2.0 g).
¹H NMR (δ, 300 MHz) 0.48 (s, 3H), 1.25-2.40 (m, 24H), 3.40-3.60 (m, 2H), 3.80-4.10 (m, 4H), 4.10-4.60 (m, 4H), 4.73 (bs, 1H), 5.50-5.80 (m, 2H), 7.30-7.45 (m, 2H), 7.76 (d, J = 8.3 Hz, 2H), 9.95 (s, 1H).

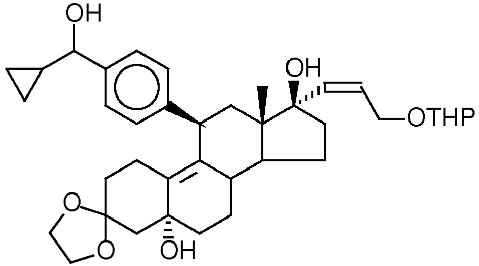

### 3,3-Ethylenedioxy-5α,17β-dihydroxy-17-(3-tetrahydropyranyloxy-prop-1(Z)-enyl)-11β-(4'-[1-cyclopropyl-hydroxymethyl] phenyl)-estr-9-ene (38)

Following the procedure outlined for the synthesis of compound **26,** the cyclopropyl addition of **37** (1.2 g) was carried out using 4 equivalents of cyclopropyl magnesium bromide and gave after workup and purification **38** (1.15 g).
¹H NMR (δ, 300 MHz) 0.25-0.70 (m, 4H), 0.50 (s, 3H), 1.25-2.50 (m, 25H), 3.25-3.60 (m, 2H), 3.80-4.10 (m, 6H), 4.15-4.60 (m, 4H), 4.73 (bs, 1H), 5.50-5.80 (m, 2H), 7.10-7.35 (m, 4H).

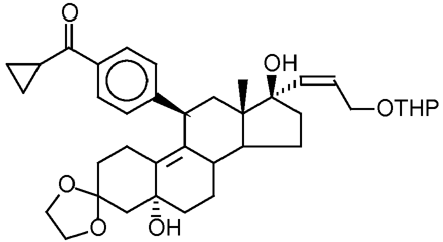

### 3,3-Ethylenedioxy-5α,17β-dihydroxy-17-(3-tetrahydropyranyloxy-prop-1(Z)-enyl)-11β-(4'-[cyclopropylcarbonyl] phenyl)-estr-9-ene (39)

Following the procedure outlined for the synthesis of compound **18,** the oxidation of **38** (1.15 g) was carried out using TPAP and NMO and gave after workup and purification **39** (1.0 g).
¹H NMR (δ, 300 MHz) 0.51 (s, 3H), 1.00-2.75 (m, 29H), 3.40-3.65 (m, 2H), 3.80-4.10 (m, 5H), 4.15-4.60 (m, 3H), 4.74 (bs, 1H), 5.50-5.80 (m, 2H), 7.26-7.40 (m, 2H), 7.92 (d, J = 8.3 Hz, 2H).

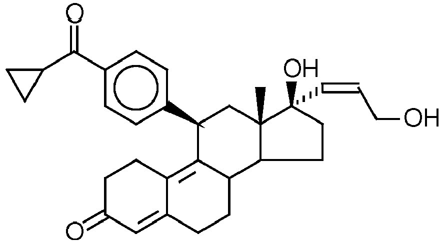

### 11β-(4'-[Cyclopropylcarbonyl]phenyl)-17β-hydroxy-17-(3-hydroxyprop-1(Z)-enyl)-estra-4,9-diene-3-one (2d) (comparative example)

Following the procedure outlined for the synthesis of compound **1a,** the hydrolysis of **39** (1.0 g) was carried out using 50% sulfuric acid to give after workup and purification **2d** (500 mg).
¹H NMR (δ, 300 MHz) 0.57 (s, 3H), 0.9-1.10 (m, 2H), 1.10-2.90 (m, 19H), 4.20-4.50 (m, 3H), 5.60-5.80 (m, 3H), 7.30 (d, J = 8.4 Hz, 2H), 7.95 (d, J = 8.4 Hz, 2H).
¹³C NMR (75 MHz) 11.7, 14.3, 15.2, 17.1, 21.1, 23.7, 25.9, 27.6, 31.1, 36.9, 38.8, 39.4, 39.5, 40.7, 47.6, 50.3, 60.5, 85.5, 123.5, 127.2, 128.5, 128.8, 130.1, 135.8, 135.9, 144.7, 150.2, 156.2, 199.2, 200.1.

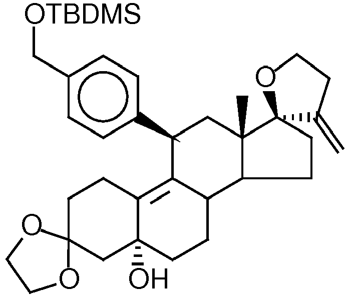

### 3,3-Ethylenedioxy-5α-hydroxy-11β-(4'-[tert-butyldimethylsilyloxymethyl]phenyl)-17,23-epoxy-19,24-dinor-17α-chola-9,20-diene (41)

¹H NMR (δ, 300 MHz) 0.08 (s, 6H), 0.51 (s, 3H), 0.93 (s, 9H), 1.0-2.7 (m, 20H), 3.6-4.1 (m, 6H), 4.1-4.2 (m, 1H), 4.70 (s, 2H), 4.82 (s, 1H), 5.08 (s, 1H), 7.1-7.4 (m, 4H).

Following the procedure outlined for the synthesis of compound **11,** the Grignard reaction of **40** (900 mg) (Jiang et al., Bioorganic and Medicinal Chemistry, 2006, 14, 6726) was carried out using 4-(t-butyldimethylsilyloxymethyl) bromobenzene and magnesium and gave after workup and purification **41** (1.4 g).

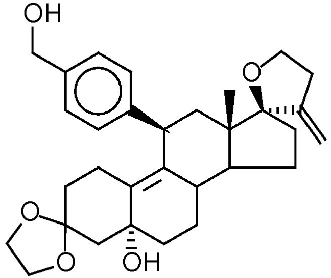

### 3,3-Ethylenedioxy-5α-hydroxy-11β-(4'-[hydroxymethyl]phenyl)-17,23-epoxy-19,24-dinor-17α-chola-9,20-diene (42)

Following the procedure outlined for the synthesis of compound **17,** the deprotection of **41** (1.4 g) was carried out using TBAF gave after workup and purification the required product **42** (700 mg).
¹H NMR (δ, 300 MHz) 0.52 (s, 3H), 0.9-2.7 (m, 20H), 3.7-4.3 (m, 7H), 4.36 (s, 1H), 4.65 (s, 2H), 4.82 (s, 1H), 5.09 (s, 1H), 7.0-7.3 (m, 4H).

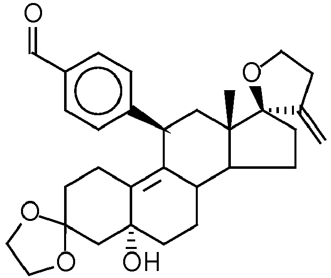

### 3,3-Ethylenedioxy-5α-hydroxy-11β-(4'-formylphenyl)-17,23-epoxy-19,24-dinor-17α-chola-9,20-diene (43)

Following the procedure outlined for the synthesis of compound **18,** the oxidation of **42** (700 mg) was carried out using TPAP and NMO and gave after workup and purification **43** (540 mg).
¹H NMR (δ, 300 MHz) 0.50 (s, 3H), 1.0-2.8 (m, 20H), 3.6-4.0 (m, 6H), 4.2-4.3 (m, 1H), 4.38 (s, 1H), 4.83 (s, 1H), 5.1 (s, 1H), 7.38 (d, J = 8.2 Hz, 2H), 7.78 (d, J = 8.3 Hz, 2H), 9.96 (s, 1H).

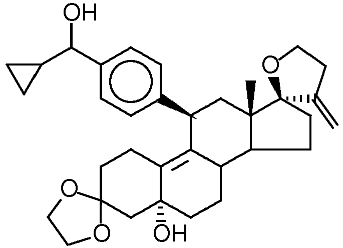

### 3,3-Ethylenedioxy-5α-hydroxy-11β-(4'-[1-cyclopropyl-hydroxymethyl]phenyl)-17,23-epoxy-19,24-dinor-17α-chola-9,20-diene (44)

Following the procedure outlined for the synthesis of compound **26,** the cyclopropyl addition of **43** (540 mg) was carried out using 4 equivalents of cyclopropyl magnesium bromide and gave after workup and purification **44** (450 mg).
¹H NMR (δ, 300 MHz) 0.51 (s, 3H), 0.2-0.7 (m, 4H), 1.0-2.7 (m, 21H), 3.6-4.0 (m, 6H), 4.1-4.2 (m, 1H), 4.37 (s, 1H), 4.82 (s, 1H), 5.09 (s, 1H), 7.17 (d, J = 8.0 Hz, 2H), 7.30 (d, J = 8.2 Hz, 2H).

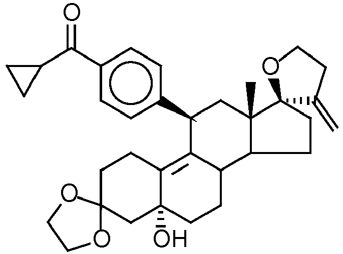

### 3,3-Ethylenedioxy-5α-hydroxy-11β-(4'-[cyclopropylcarbonyl]phenyl)-17,23-epoxy-19,24-dinor-17α-chola-9,20-diene (45)

Following the procedure outlined for the synthesis of compound **18,** the oxidation of **44** (450 mg) was carried out using TPAP and NMO and gave after workup and purification **45** (400 mg).
¹H NMR (δ, 300 MHz) 0.51 (s, 3H), 1.0-2.8 (m, 25H), 3.6-4.0 (m, 6H), 4.1-4.3 (m, 1H), 4.38 (s, 1H), 4.83 (s, 1H), 5.10 (s, 1H), 7.31 (d, J = 8.2 Hz, 2H), 7.91 (d, J = 8.2 Hz, 2H).

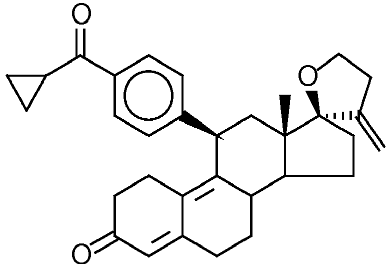

### 11β-(4'-[Cyclopropylcarbonyl]phenyl)-17,23-epoxy-19,24-dinor-17α-chola-4,9,20-triene-3-one (2e) (comparative example)

Following the procedure outlined for the synthesis of compound **1a,** the hydrolysis of **45** (400 mg) was carried out using 50% sulfuric acid to afford **2e** (350 mg) after workup and purification.
¹H NMR (δ, 300 MHz) 0.57 (s, 3H), 0.90-1.30 (m, 4H), 1.30-2.80 (m, 19H), 3.70-390 (m, 2H), 4.35 (d, J = 7.4 Hz, 1H), 4.86 (s, 1H), 5.15 (s, 1H), 5.78 (s, 1H), 7.28 (d, J = 8.4 Hz, 2H), 7.95 (d, J = 8.4 Hz, 2H).
¹³C NMR (75 MHz) 11.56, 11.59, 15.2, 17.0, 23.7, 25.81, 27.5, 31.1, 34.2, 35.01, 36.7, 39.08, 39.9, 40.8, 46.8, 49.01, 59.5, 64.8, 94.5, 107.3, 123.3, 127.2, 128.4, 129.7, 135.8, 144.9, 150.32, 154.1, 156.2, 199.1, 200.0.

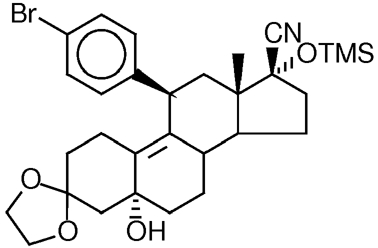

### 3,3-Ethylenedioxy-17β-cyano-5α-hydroxy-17α-trimethylsilyloxy-11β-[4'-bromophenyl]-estr-9-ene (46)

Following the procedure outlined for the synthesis of compound **11,** except the Grignard was formed at rt by stirring 1,4-dibromobenzene and magnesium at rt for 3hrs. Reaction was carried out using epoxide **21** (5.15 g) and gave after workup and purification **46** (6 g).
¹H NMR (δ, 300 MHz) 0.24 (s, 9H), 0.51 (s, 3H), 1.0-2.5 (m, 18H), 3.8-4.0 (m, 4H), 4.3 (m, 1H), 4.45 (s, 1H), 7.1 (d, J = 8.5 Hz, 2H), 7.38 (d, J = 8.5 Hz, 2H).

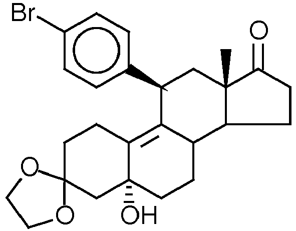

### 3,3-Ethylenedioxy-5α-hydroxy-11β-[4'-bromophenyl]-estr-9-ene-17-one (47)

Following the procedure outlined for the synthesis of compound **23,** the deprotection of **46** (6.5 g) was effected using TBAF/NaOH and gave after workup and purification **47** (5.2 g).
¹H NMR (δ, 300 MHz) 0.48 (s, 3H), 1.0-2.6 (m, 18H), 3.8-4.0 (m, 4H), 4.26 (d, J = 7.3 Hz, 1H), 4.38 (s, 1H), 7.10 (d, J = 8.4 Hz, 2H), 7.37 (d, J = 8.5 Hz, 2H).

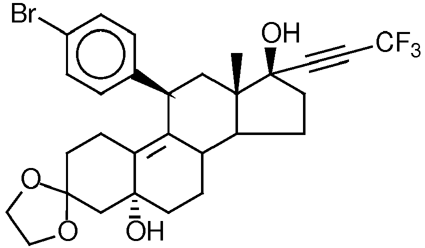

### 3,3-Ethylenedioxy-5α,17β-dihydroxy-17-(3,3,3-trifluoro-1-propynyl)-11β-[4'-bromophenyl]-estr-9-ene (48)

Following the procedure outlined for the synthesis of compound **6,** the addition of trifluoropropyne to **47** (1.0 g) was carried out using 2-bromo-3,3,3-trifluoropropene and LDA at - 78° C and gave after workup and purification **48** (1.0 g).
¹H NMR (δ, 300 MHz) 0.48 (s, 3H), 1.0-2.8 (m, 18H), 3.8-4.2 (m, 4H), 4.3 (bs, 1H), 4.42 (s, 1H), 7.09 (d, J = 8.4 Hz, 2H), 7.37 (d, J = 8.5 Hz, 2H).

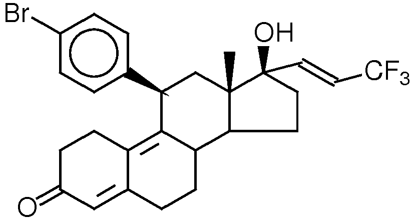

### 11β-(4'-Bromophenyl)-17β-hydroxy-17-(3,3,3-trifluoroprop-1(E)-enyl)-estra-4,9-diene-3-one (49)

Following the procedure outlined for the synthesis of compound **7,** the Red-Al reduction of **48** (1.0 g) was carried out at -78° C and the crude product obtained after workup was subjected to hydrolysis using the procedure outlined for **1a.** Reaction after workup and purification using 20% ethyl acetate in hexane afforded **50** (750 mg).
¹H NMR (δ, 300 MHz) 0.59 (s, 3H), 1.2-2.8 (m, 16H), 4.32 (d, J = 7.2 Hz, 1H), 5.78 (s, 1H), 5.95 (dq, J = 15.5, 6.5 Hz, 1H), 6.53 (dd, J = 2, 15.5 Hz, 1H), 7.03 (d, J = 8.3 Hz, 2H), 7.39 (d, J = 8.5 Hz, 2H).

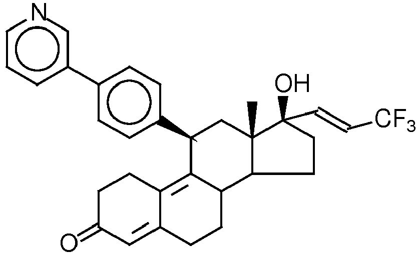

### 11β-(4'-[3-Pyridinyl]phenyl)-17β-hydroxy-17-(3,3,3-trifluoroprop-1(E)-enyl)-estra-4,9-diene-3-one (3a)

A mixture of 11β-(4'-bromophenyl)-17β-hydroxy-17-(3,3,3-trifluoroprop-1(*E*)-enyl)-estra-4,9-diene-3-one (**50**) (500 mg), 3-pyridinylboronic acid (295 mg), bis (triphenylphosphine) palladium (II) chloride (34 mg) and triphenylarsine (35 mg) were taken in dioxane (24 mL). The reaction mixture was degassed by applying light vacuum followed by filling with argon and finally an aqueous solution of potassium carbonate (200 mg in 4.2 mL) was introduced. The reaction mixture was again degassed and allowed to reflux for 4 hrs at 100° C. Reaction was cooled to rt and diluted with water (20 mL) and organic material were extracted with ethyl acetate (3X20 mL). The combined organic layer was washed once with brine (15 mL), dried over sodium sulfate, concentrated under reduced pressure and purified on a silica gel using 50% ethyl acetate in hexane to afford **3a** (400 mg).
¹H NMR (δ, 300 MHz) 0.64 (s, 3H), 1.2-2.9 (m, 16H), 4.43 (d, J = 7.2 Hz, 1H), 5.80 (s, 1H), 6.00 (dq, J = 15.5, 6.5 Hz, 1H), 6.57 (dd, J = 2, 15.5 Hz, 1H), 7.26 (d, J = 8.3 Hz, 2H), 7.3-7.4 (m, 1H), 7.50 (d, J = 8.2 Hz, 2H), 7.87 (d, J = 7.9 Hz, 1H), 8.57 (d, J = 4.7 Hz, 1H), 8.82 (d, J = 2.2 Hz, 1H).

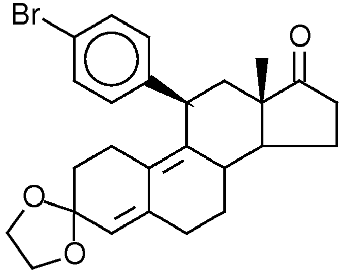

### 3,3-Ethylenedioxy-11β-(4'-bromophenyl)-estra-4,9-diene-17-one (51)

Following the procedure outlined for the synthesis of compound **12,** the dehydration **47** (2.0 g) was carried out using acetic anhydride and pyridine and gave after purification **51** (1.5 g)
¹H NMR (δ, 300 MHz) 0.49 (s, 3H), 1.2-2.8 (m, 16H), 3.7-4.0 (m, 4H), 4.2 (m, 1H), 5.40 (s, 1H), 7.1 (d, J = 8.0 Hz, 2H), 7.37 (d, J = 8.2 Hz, 2H).

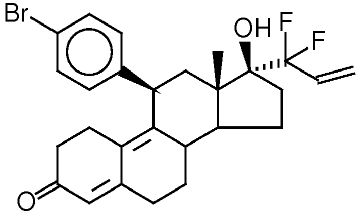

### 11β-(4'-Bromophenyl)-17β-hydroxy-17-(1,1-difluoroprop-2-enyl)-estra-4,9-diene-3-one (52)

Following the procedure outlined for the synthesis of compound **1d,** the difluoropropene addition to **51** (1.05 g), followed by acid hydrolysis yielded **52** (350 mg).
¹H NMR (δ, 300 MHz) 0.57 (s, 3H), 1.2-2.8 (m, 16H), 4.3 (m, 1H), 5.55 (d, J = 11 Hz, 1H), 5.73 (d, J = 17.5 Hz, 1H), 5.77 (s, 1H), 6.0-6.3 (m, 1H), 7.06 (d, J = 8.1 Hz, 2H), 7.39 (d, J = 8.5 Hz, 2H).

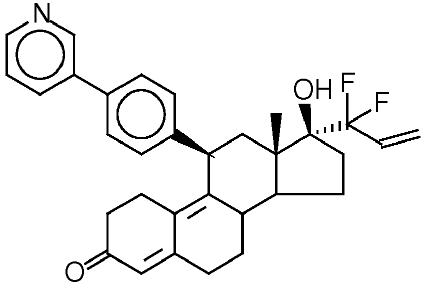

### 11β-(4'-[3-Pyridinyl]phenyl)-17β-hydroxy-17-(1,1-difluoroprop-2-enyl)-estra-4,9-diene-3-one (3b)

Following the procedure outlined for the synthesis of **3a,** the Suzuki coupling of **52** (350 mg) yielded **3b** (100 mg).
¹H NMR (δ, 300 MHz) 0.62 (s, 3H), 1.2-2.8 (m, 16H), 4.47 (bs, 1H), 5.56 (d, J = 11 Hz, 1H), 5.74 (d, J = 17.4 Hz, 1H), 5.8 (s, 1H), 6.1-6.3 (m, 1H), 7.10-7.27 (m, 2H), 7.35 (dd, J = 8,4 Hz, 1H), 7.4-7.5 (m, 2H), 7.84 (dt, J = 8,1 Hz, 1H), 8.57 (dd, J = 4.8,1.5 Hz, 1H), 8.82 (d, J = 1.8 Hz, 1H).

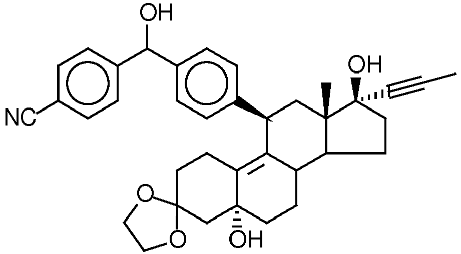

### 3,3-Ethylenedioxy-5α,17β-dihydroxy-17-(1-propynyl)-11β-(4'-[1-{4-cyanophenyl}-hydroxymethyl] phenyl)-estr-9-ene (53)

A solution of 4-bromobenzonitrile (2.67g, 14.68 mmol) in dry THF (10mL) was cooled to -8°C in an ice-salt bath, a solution of isopropyl magnesium chloride-Lithium chloride complex in THF (1.3 M, 11.3 mL, 14.68 mmol) was added dropwise over a period of 20 min. The resulting yellow colored solution was stirred at 0°C for 3h. A solution of **25** (1.4g, 2.93 mmol) in dry THF (10 mL) was added dropwise and stirred for 2h. The reaction was found to be complete at this time and was quenched by adding sat.NH₄Cl solution (10mL). The reaction mixture was extracted using ethyl acetate (2x50 mL). The combined extracts were washed with water (100mL), brine (100mL) and dried over Na₂SO₄. The solvent was removed in *vacuo* and the crude (2.78g) was purified by flash column chromatography (SiO₂ 80:20 Hexane:EtOAc) to afford 1.44g of **53** as a white fluffy solid in 85% yields.
¹H NMR (δ, 300 MHz) 0.39 (s, 3H), 1.86 (s, 3H), 3.90-3.98 (m, 4H), 4.30 (d, J = 7.0 Hz, 1H), 4.43 (s, 1H), 5.80 (s, 1H), 7.15-7.19 (m, 4H), 7.48 (d, J = 8.1 Hz, 2H), 7.61 (d, J = 8.1 Hz, 2H). ¹³C NMR (75 MHz) 3.76, 13.58, 23.16, 23.54, 23.94, 34.95, 38.17, 38.90, 38.97, 46.57, 47.29, 49.28, 59.36, 63.97, 64.60, 70.08, 75.21, 80.13, 82.20, 82.36, 108.57, 110.94, 111.0, 118.77, 126.43, 126.50, 126.93, 126.98, 127.55, 127.57, 132.13, 132.16, 133.99, 134.31, 139.80, 139.83, 147.13, 147.19, 149.0.

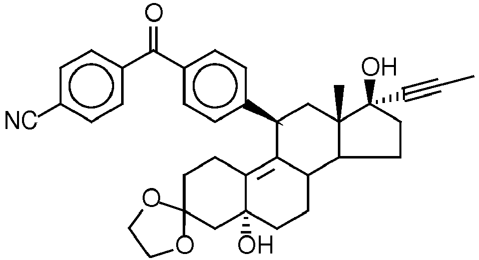

### 3,3-Ethylenedioxy-5α,17β-dihydroxy-17-(1-propynyl)-11β-(4'-[4-cyanobenzoyl]phenyl)-estr-9-ene (54)

Following the procedure outlined for the synthesis of compound **18,** the oxidation of **53** (1.4 g) was carried out using TPAP and NMO and gave after workup and purification **54** (1.15 g).
¹H NMR (δ, 300 MHz) 0.45 (s, 3H), 1.88 (s, 3H), 3.92-4.0(m, 4H), 4.41 (d, J=7.1 Hz, 1H), 4.46 (s, 1H), 7.39 (d, J=8.4 Hz, 2H), 7.71 (d, J=8.4 Hz, 2H), 7.78-7.87 (m, 4H).

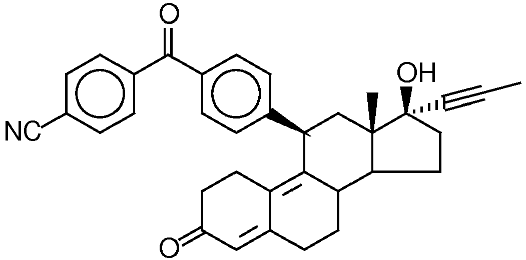

### 11β-(4'-[4-Cyanobenzoyl]phenyl)-17β-hydroxy-17-(1-propynyl)-estra-4,9-diene-3-one (4) (comparative example)

Following the procedure outlined for the synthesis of compound **1a,** the hydrolysis of **54** (1.15 g) was carried out using 50% sulfuric acid to give after workup and purification **4** (830 mg)
¹H NMR (δ, 300 MHz) 0.52 (s, 3H), 1.91 (s, 3H), 4.51 (d, J=6.9 Hz, 1H), 4.46 (s, 1H), 5.80 (s, 1H), 7.3 (d, J=8.2 Hz, 2H), 7.73 (d, J=8.2 Hz), 7.77-7.87 (m, 4H).
¹³C NMR (75 MHz) 3.79, 13.86,14.16, 21.01, 23.30, 25.91, 27.32, 31.03, 36.72, 38.89, 39.20, 40.72, 46.87, 49.57, 60.35,79.97, 82.04, 82.83, 115.53, 117.98, 123.40, 127.32,130.07, 130.52, 133.90, 141.35, 144.34, 151.26, 156.07, 194.36, 199.03.

### Example 1

### Nuclear Receptor Profiling

Determination of the agonist/antagonist nature of the test compounds was carried out using Invitrogen's SelectScreen™ Cell-based nuclear receptor profiling service which uses the GeneBLAzer® Beta-lactamase reporter technology. Basically this assay uses a Beta-lactamase cDNA under transcriptional control of an Upstream Activator Sequence (UAS). The UAS is activated by the GAL4 transcription factor DNA binding domain (DBD), which is expressed as a fusion protein with the target receptor ligand binding domain (LBD). Upon ligand binding, the GAL4(DBD)-NR(LDB) binds to the UAS, which controls transcription of Beta-lactamase. Beta-lactamase cleaves a special engineered fluorescent substrate which results in a change in the measured fluorescence wavelength.

The generalized protocol used for the Progesterone Antagonist Screen, activated by control Agonist R5020 is as follows:
The progesterone receptor-LBD-UAS-bla HEK 293T cells are thawed and prepared as described above for the Agonist screen. 4 µL of a 10X serial dilution of control antagonist RU-486 (starting concentration, 100 nM) or test compounds are added to appropriate wells of a TC-Treated assay plate. 32 µL of cell suspension is added to the wells which is then pre-incubated at 37 °C/5% CO2 in a humidified incubator with test compounds and control antagonist titration for 30 minutes. 4 µL of a 10X control agonist (see above) at the predetermined EC80 concentration is added to wells containing the control antagonist or test compounds. The plate is incubated for 16-24 hours at 37 °C/5% CO2 in a humidified incubator. 8 µL of 1 µM Substrate Loading Solution is added to each well and the plate is incubated for 2 hours at room temperature. The plate is then read on a fluorescence plate reader (Tecan Safire).

The generalized protocol for the Glucocorticoid Antagonist Screen activated by control Agonist Dexamethasone was carried out as described for the Progesterone Antagonist Screen with the exception that glucocorticoid receptor-LBD-UAS-bla HEK 293T cells were used. The control antagonist used for the glucocorticoid assay was also RU-486.

The results of these tests for the indicated test compounds are shown in Table I

**Table I**

| **Compounds** | **Progesterone Antagonist (%)** | **Glucocorticoid Antagonist (%)** |
|---|---|---|
| **ZK 230211** | 81 | 6 |
| **1a** | 6 | NA |
| **1b** | 33 | 22.1 |
| **1c** | 21 | 6 |
| **1d** | 119 | 8.3 |
| **1e** | 191 | 10 |
| **2a** | 91 | 16.7 |
| **2b** | 45 | 40 |
| **2c** | 2 | NA |
| **2d** | 6 | NA |
| **2e** | 48 | 2 |
| **3a** | 190 | 52 |
| **3b** | 200 | 31 |
| **4** | 38 | 38 |

| | | |
|---|---|---|
| NA = not available; values are given in relative to RU486, which is 100% | | |

### Example 2

### Antinidation Tests in Rat

For every compound to be screened in rats (Sprague-Daley) 3 control rats (vehicle treated, s.c.), 3 rats treated (s.c.) with a known progestin antagonist and 3 rats treated (s.c.) with the test compound (3 mg/day) are used. Female rats will be placed with male rats for 3 to 4 days and exam the vagina for sperm plugs every morning. The presence of a sperm plug will indicate day 1 of pregnancy. Pregnant rats will be treated daily with 3 mg of the screened compound at beginning on day 5 of pregnancy. At day 9 of gestation the rats will be euthanized and nidation sites counted.

| **Compounds** | **Implantation Sites*** |
|---|---|
| **Vehicle** | **3/3** |
| **CDB-4124** | **0/3** |
| **ZK98299** | **2/3** |
| **1a** | **1/3** |
| **1b** | **0/3** |
| **1c** | **0/3** |
| **2a** | **0/3** |
| **3a** | **0/4** |
| **3b** | **0/4** |

| | |
|---|---|
| *0/3 Refers to full progesterone antagonist and none of the 3 rats had any implantation sites. | |

### Example 3

### Invitro Antiproliferative Activity in Human Breast Cancer Cell Line T47D

Cells from frozen stock were expanded in T75 cell culture flasks. For the experiment, cells in the logarithmic growth phase (70 % confluent) were cultivated for 3 day without estradiol and then washed with PBS, detached by trypsination and suspended in 5 ml of fresh RPMI-1640 medium. Cells were centrifuged for 5 min at 1000 rpm and the pellet was resuspended in RPMI-1640 medium.

Cells were seeded in 96-well plates with a density of 5000 cells/well/ in 0.18 mL RPMI-1640 medium. Cells were allowed to attach for 24 h. Visual control for viability at 24h and cell culture medium change. At this time point, compounds were added in 20µl to the final concentration. After 3 days medium and compounds where changed again. Finally, 7 days after cell seeding, 20µl MTT-solution was added to each well and 4h later the formed tetrazolium salt was measured with a photometer.

| **Compounds** | **IC50 (nM)** |
|---|---|
| **ZK230211** | **0.3** |
| **1a** | **17** |
| **1b** | **0.64** |
| **1c** | **3.1** |
| **1d** | **0.3** |
| **1e** | **0.3** |
| **2a** | **1.2** |
| **2b** | **0.64** |
| **2c** | **4.6** |
| **2e** | **0.37** |

### Example 4

### Uterine Weights of Guinea Pigs

3 Non-pregnant guinea pigs in the luteal phase of the cycle will be treated with 10 mg of the compound subcutaneously beginning on day 10 of the cycle. Animals were sacrificed on day 18 after the start of the treatment and the uterine weights were obtained at the time of sacrifice. Comparisons will be made to onapristone-treated animals at 10 mg/animal/day s.c

| **Compounds** | **Uterine Weights of Guinea Pigs Treated with antiprogestins (grams)*** |
|---|---|
| **ZK98299** | **1.07** |
| **CDB 4124** | **1.41** |
| **RU 486** | **1.81** |
| **1a** | **1.3** |
| **1b** | **0.785** |
| **1c** | **1.8** |
| **2a** | **2.3** |
| **2c** | **1.35** |
| **2d** | **0.99** |
| **2e** | **1.02** |
| **3a** | **0.87** |

| | |
|---|---|
| * Uterine weight of untreated guinea pig was 1.2 g, uterine weights of a value higher than 1.2 g indicate that the test compounds exhibit pure progesterone antagonistic activity and subsequently unopposed estrogenicity, that lead to growth stimulation of the uterine tissue. | |

## Claims

1. A compound having the structure: 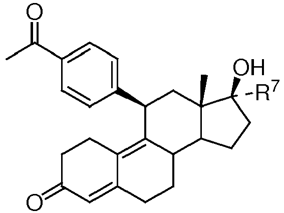 wherein R⁷ is (E)-CH=CH-CF₃, -CH₂-CF=CF₂, or -CF₂-CH=CH₂.

2. A compound having the structure: 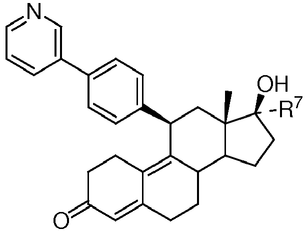 wherein R⁷ is (E)-CH=CH-CF₃ or -CF₂-CH=CH₂.

3. A compound as described in claim 1 or 2 for use in the treatment of cancer in a subject.

## Patentansprüche

1. Verbindung mit der Struktur: 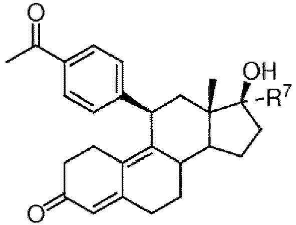 wobei R⁷ für (E) -CH=CH-CF₃, -CH₂-CF=CF₂ oder -CF₂-CH=CH₂ steht.

2. Verbindung mit der Struktur: 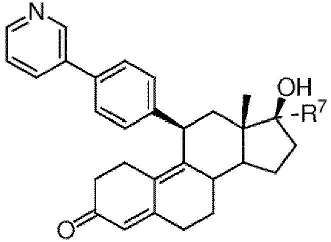 wobei R⁷ für (E) -CH=CH-CF₃ oder -CF₂-CH=CH₂ steht.

3. Verbindung nach Anspruch 1 oder 2 zur Verwendung bei der Behandlung von Krebs bei einem Patienten.

## Revendications

1. Composé présentant la structure : 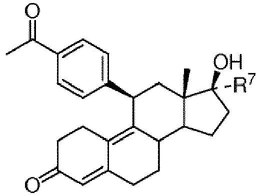 dans laquelle R⁷ est (E) -CH=CH-CF₃, -CH₂-CF=CF₂ ou-CF₂-CH=CH₂.

2. Composé présentant la structure : 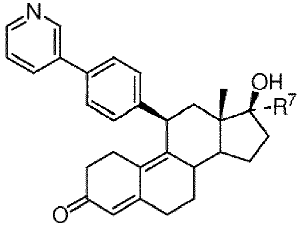 dans laquelle R⁷ est (E)-CH=CH-CF₃ ou -CF₂-CH=CH₂.

3. Composé tel que décrit dans la revendication 1 ou 2, destiné à une utilisation dans le traitement du cancer chez un sujet.
